(19) Europäisches Patentamt
European Patent Office
Office européen des brevets

(11) Publication number: **0 118 223**
**A1**

# (12) EUROPEAN PATENT APPLICATION

(21) Application number: 84300733.7

(22) Date of filing: 06.02.84

(51) Int. Cl.$^3$: A 61 K 37/64
C 07 C 103/52

(30) Priority: 07.02.83 US 469540
19.08.83 GB 8322414

(43) Date of publication of application:
12.09.84 Bulletin 84/37

(84) Designated Contracting States:
AT BE CH DE FR GB IT LI LU NL SE

(71) Applicant: Szelke, Michael
10 North Drive
Ruislip Middlesex(GB)

(71) Applicant: Jones, David Michael
89 Woodrow Avenue
Hayes Middlesex(GB)

(71) Applicant: Hallet, Allen
60 Kingsdown Road
Cheam Surrey(GB)

(71) Applicant: Atrash, Butrus
9 Testwood Court, Golden Manor
Hanwell London W.7(GB)

(72) Inventor: Szelke, Michael
10 North Drive
Ruislip Middlesex(GB)

(72) Inventor: Jones, David Michael
89 Woodrow Avenue
Hayes Middlesex(GB)

(72) Inventor: Hallet, Allen
60 Kingsdown Road
Cheam Surrey(GB)

(72) Inventor: Atrash, Butrus
9 Testwood Court, Golden Manor
Hanwell London W.7(GB)

(74) Representative: Farwell, William Robert et al,
PHILLIPS & LEIGH 7 Staple Inn Holborn
London WC1V 7QF(GB)

## (54) Enzyme inhibitors.

(57) Renin-inhibiting tetra-, penta- or hexapeptide analogues of formula

X–D–E——A——B–Z–W (XII)
8 9 10, 11 12 13

where X and W are terminal groups; D, E, B and Z (of which any one or except with 'reduced' analogues any two may be absent) are aromatic, lipophilic or in the case of E aromatic lipophilic or basic amino acid or amino acid analogue residues; and A is an analogue of a lipophilic or aromatic dipeptide residue wherein the peptide link is replaced by a one- to four-atom carbon or carbon-nitrogen link which as such or in hydrated form is an unhydrolysable tetrahedral analogue of the transition state

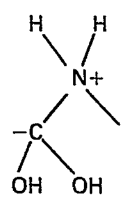

of the peptide bond.

EP 0 118 223 A1

ENZYME INHIBITORS

The invention relates to enzyme inhibitors, and particularly renin-inhibiting peptide analogues.

BACKGROUND

Renin is a natural enzyme, disorders in relation to which are implicated in many cases of hypertension. It is released into the blood from the kidney, and cleaves from a blood glycoprotein a decapeptide known as angiotensin-I. Circulating angiotensin-I is cleaved in lung, kidney and other tissues to an octapeptide, angiotensin-II, which raises blood pressure both directly by causing arteriolar constriction and indirectly by stimulating release of the sodium-retaining hormone aldosterone from the adrenal gland and thus causing a rise in extracellular fluid volume. The latter effect is caused by angiotensin-II itself or a heptapeptide cleavage product angiotensin-III.

Inhibitors of renin have therefore been sought, with two ends in view, first the provision of a diagnostic agent for identification of cases of hypertension due to renin excess, and secondly the provision of an agent for control of hypertension in such cases.

The present inventors' approach has been to consider the peptide sequence characterising the natural renin substrate at its binding site, and to seek peptide analogues sufficiently similar to bind to the enzyme, in competition with the natural substrate, but sufficiently dissimilar to it to be cleaved slowly or not at all.

Such analogues will block the action of the enzyme and attack the hypertension at source.

Renin is specific to a particular bond in the substrate, the N-terminal sequence of which in the horse is for example: (IA)

```
  C                                        B         A
  ↓                                        ↓         ↓
Asp-Arg-Val-Tyr-Ile-His-Pro-Phe-His-Leu-Leu-Val-Tyr-Ser-
 1   2   3   4   5   6   7   8   9   10  11  12  13  14
```

as found by L.T. Skeggs et al J. Exper. Med. 106 439 (1957). Human renin substrate has a different sequence recently discovered by D.A. Tewkesbury et al Biochem. Biophys. Res. Comm. 99 1311 (1981).

```
A                                                  (IB)
↓
-Val-Ile-His-      .....
 11  12  13
```

the sequence to the left of the arrow A being as in formula (IA).

Cleavage at A gives angiotensin-I; subsequent cleavage at the Phe-His bond at B gives angiotensin-II; and cleavage subsequently again at the Asp-Arg bond at C gives angiotensin-III.

Peptides similar to certain partial sequences of the substrate have been shown to act as inhibitors of renin in vitro. An example is the tetrapeptide ester (the relation to the substrate residues being indicated by numbering):

```
H-Leu-Leu-Val-Phe-OMe                              (II)
  10  11  12  13
```

proposed by Kokubu, Nature, 217 456 (1968) but it is inactive in vivo, because of binding to plasma proteins and rapid attack by natural peptidases.

One of the present inventors undertook some years ago a development of Kokubu's work, seeking a renin inhibitor active in vivo, in which analogues of peptides similar to Kokubu's were made but having a methylene imino group $-CH_2-NH-$ in place of the peptide link $-CO-NH-$ between the leucine residues. One of these analogues was:

$$\underset{\substack{|\\ NH_2-CH}}{{}^{i}Bu}-CH_2-NH-\underset{\substack{|\\ CH}}{{}^{i}Bu}-CO-Val-Phe-OMe \qquad (III)$$

10 11 12 13

which is the tetrapeptide (I) modified at the Leu-Leu link, leucine of course being

$$\underset{\substack{|\\ NH_2-CH}}{{}^{i}Bu}-COOH \qquad (IV)$$

This analogue (III) was the first effective in-vivo inhibitor of renin and was shown to have significant antihypertensive action in Goldblatt hypertensive rats (Parry, Russell and Szelke p. 541 in "Chemistry and Biology of Peptides" Ed. Meienhofer, Ann Arbor Science Publishers 1972). Little or no attention was however paid to the work, which the authors themselves were unable to pursue, in spite of considerable activity in the general field of substrate-based inhibitors for renin, reviewed for example by Haber & Burton, Federation Proc. 38 No. 13 2768-2773 (1979).

## BASIS OF INVENTION

The invention is based on recognition of the fact that renin has a much greater binding affinity for the transition-state than for the substrate. Non-hydrolysable analogues of the transition-state VI (Table I below) formed at the scissile peptide bond V during

hydrolysis of the substrate therefore provide potent enzyme inhibitors.

Table I

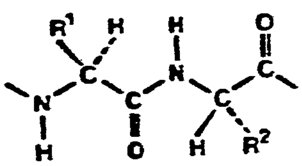

Peptide V

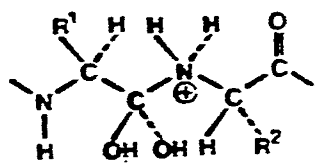

Transition state VI

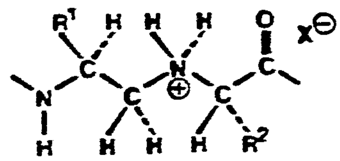

Reduced analogue VII

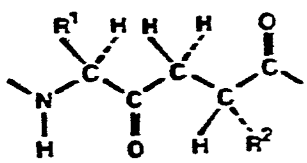

Keto analogue VIII

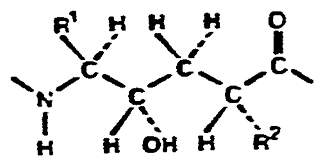

Hydroxy analogue IX

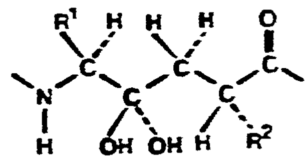

Hydrated keto analogue X

($R^1$, $R^2$ in this table = amino acid side chain(s))

Thus the inventors have recognised that replacement of the scissile peptide bond V, in a partial sequence or partial sequence analogue of renin substrate, with a 'reduced' analogue VII, a hydroxy analogue IX, the novel 'amino' analogue $-CH(NH_2)-CH_2-$ (compare IX) or the 'hydrocarbon' analogue $-CH_2-CH_2-$ (again compare IX) provides such non-hydrolysable analogues. They have also recognised that the 'keto' analogue VIII is capable of reacting with the water molecule present at the active site of aspartic proteinases such as renin to form a tetrahedral hydrate X which is a further non-hydrolysable transition-state analogue. These analogues are also referred to herein as isosteres, and the links they give

in the backbone as isosteric links.

Based on structure-activity studies in their own laboratory, and on recent x-ray crystallographic studies (e.g. Blundell et al Nature 304 273 (1983) the inventors have devised novel cyclic structures and other modifications of the substrate sequence including the backbone and N- and C-terminal positions which both increase binding affinity to renin and impart greater stability against breakdown by exo- and endo- peptides in vivo.

In particular, a study of the enzyme-inhibitor complex by computer graphics suggested, and experiments have confirmed, that replacing the hydroxyl function of the 'hydroxy' isostere IX with an amino group capable of carrying a positive charge ('amino' isostere) introduces additional binding through interaction with the negative charge of the aspartic carboxyl functions at the active site of the enzyme. Replacing the hydroxyl group of statine with an amino group to give 'amino-deoxy-statine' has a similar effect.

A particular aspect of the present invention stems from a desire to provide orally active renin inhibitors. On the one hand, a shorter sequence and increased lipophilicity favour absorption from the gut. On the other hand, a longer sequence is usually required to maintain high inhibitory potency and selectivity for renin as opposed to other, related proteases in the body. The inventors have found that a balance of these requirements can be achieved by a suitable combination of molecular parameters. For example, the introduction

of lipophilic groups can more than offset the loss of potency due to a shortening of the peptide sequence, and at the same time facilitate absorption and increase stability against enzymatic breakdown in vivo (e.g. compare H-270 with H-269, 287 and 288 in Table 2 later herein).

Generally the invention uses a concept of modifying peptide structures related to the peptide sequence at the site of action of renin on the natural substrate, by isosteric substitution at, at least, the site of cleavage. Optionally further there is isosteric substitution or other modification at other positions to increase stability or to modify the properties of the final peptide, for example its solubility under physiological conditions or its resistance to in vivo exopeptidase attack. Such modification may for example be by incorporation of residues other than those of the natural L-amino acids; by protection of the N-terminus with acetyl, pivaloyl, t-butyloxycarbonyl (Boc), benzoyl or other groups; or by conversion of the C-terminal carboxyl to another functional group, e.g. the corresponding alcohol, present as such or in ether or ester form.

INVENTORS' PREVIOUS PATENT APPLICATIONS

Some of the above ideas have been applied in an earlier application of the inventors related to the present and published as European Patent Specification A 0 045 665 (application No. 81 3 03585.4). In that specification compounds are disclosed of the general formula:

X-Y-Pro-Phe-His——A——B—Z—W (XI)

6 7 8 9 10,11 12 13

where X and W are various terminal groups, Y (which is optional) is His or other basic or aromatic amino-acyl residue, A is a 'reduced', 'keto', 'hydroxy' or 'hydrocarbon' isostere of a dipeptide, B is a lipophilic amino acyl residue and Z is an aromatic amino acyl residue.

Some of the ideas also appear, in a different context, in unpublished European application 83 3 05353.1.

DEFINITIONS

The following definitions apply both to the description of the invention and to the claims unless otherwise specified:

(1) The term 'amino acid' also includes imino acids (e.g. proline, spinacin); furthermore, it includes amino acids of both natural and unnatural origin.

(2) All amino acids may be of either L- or D-configuration unless stated otherwise.

(3) All asymmetric centres may be of either R or S configuration, unless stated otherwise.

(4) The term 'alkyl' includes both branched and straight chain hydrocarbon groups having 1-6 carbon atoms.

(5) The term 'aryl' (abbreviated 'Ar') means phenyl or other (including mono- or bicyclic) aromatic groups, which may be substituted, especially monosubstituted, with one of the following groups, preferably (when phenyl) in the 2- or 4-position

F, Cl, Br, I, $-CF_3$, -OH, -OR or -R (R = alkyl)

(6) The term 'aromatic amino acyl' defines amino acid residues having as side-chain an aryl-methyl ($ArCH_2$), imidazol-4-yl-methyl or indol-3-yl-methyl group.

Reference to basic and aromatic amino acids above, and to amino acids with lipophilic side chains, in particular includes but is not restricted to the common amino acids of those classes, viz:

| | | |
|---|---|---|
| Basic: | Arginine | |
| | Lysine | |
| | Histidine | |
| Aromatic: | Phenylalanine | |
| | Tyrosine | |
| | Tryptophan | |
| | Histidine | |
| | αNal | ) unnatural |
| | β Nal | ) |
| Lipophilic: | Leucine | |
| | Isoleucine | |
| | Valine | |
| | Phenylalanine | |
| | Cyclohexylalanine | ) unnatural |
| | Adamantylalanine | ) |

It should be noted that symbols X, Y, A etc. in formulae (XI), (XII), (XIV) and (XV) below, while generally equivalent where the same symbol is used, are each as defined for their individual formula both in the description and in the claims.

THE PRESENT INVENTION

The present invention is most briefly stated as providing in its main aspect renin-inhibiting tetra-, penta- or hexapeptide analogues of formula

X-D-E——A——B—Z—W (XII)

8 9 10,11 12 13

where X and W are terminal groups; D, E, B and Z, of which any one or, except with 'reduced' analogues, two may be absent, are aromatic, lipophilic or (in the case of E) aromatic, lipophilic or basic amino acid or amino acid analogue residues; and A is an analogue of a lipophilic or aromatic dipeptide residue wherein the peptide link is replaced by a one- to four-atom carbon or carbon-nitrogen link which as such or in hydrated form is an unhydrolysable tetrahedral analogue of the transition state of the peptide bond as given above. (The numbering above and in other formulae herein indicates the relation to the natural renin substrate sequence, though without limitation of the invention).

The invention extends further to sundry novel dipeptide analogues with the replaced peptide link, both as such and when used in renin-inhibitory or other peptide analogues of any length, particularly 'amino', 'cyclic' and 'aminostatine' compounds as referred to herein.

Finally the invention extends to the new compound 3-amino-3-deoxy-statine ('amino-statine')

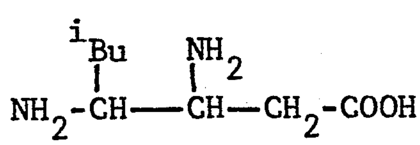

(XIII)

which is 3,4-diamino-6-methyl-heptanoic acid.

In more detail the compounds of the present invention are of the general formula

X—D—E——A——B—Z—W (XIV)

8 9 10,11 12 13

where

X = H or an N-protecting group or groups, e.g. as follows:

(a) $R^3-(CH_2)_n-$, where n = 0-4, or

(b) $R^3-CO-$ or

(c) $R^3-O-CO-$ or

(d) $R^3-(CH_2)_n-CO-$ or )

(e) $R^3-(CH_2)_n-O-CO$ or ) where n = 0-5

(f) $R^3-O-(CH_2)_n-CO-$ or )

(g) $R^3SO_2-$ or

(h) $(R^3)_2-N-CO-$

In (a)-(h), $R^3$ = (i) H (except in (c) ) or

(ii) alkyl (e.g. t-butyl) or

(iii) cycloalkyl $C_{3-7}$ (e.g. cyclohexyl) or

(iv) bicycloalkyl or tricycloalkyl $C_{7-12}$ (e.g. isobornyl or adamantyl) or

(v) aryl (e.g. phenyl) or aryl alkyl

D = (a) absent

(b) aromatic amino acyl (e.g. Phe, αNal, βNal, Tyr, His, Trp)

(c) lipophilic amino acyl (e.g. cyclohexyl-alanyl)

(with (b) and (c) either as such or reduced at carbonyl)

E = (a) absent or

(b) aromatic amino acyl (e.g. His, Phe, Tyr, Trp) or

(c) lipophilic or basic amino acyl (e.g. 2-aminobutyryl, α, δ -diaminovaleryl), (b) and (c) being as such or $N^{\alpha}$ -alkylated and/or reduced at carbonyl

A = (a) 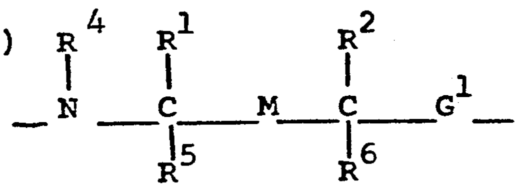 where

$R^4$, $R^5$ and $R^6$ the same or different =

(i) H or

(ii) alkyl (e.g. Me) or

(iii) $-(CH_2)_n-OH$ or $-(CH_2)_n-NH_2$ when n = 2, 3, 4

$G^1$ (and $G^2$ appearing below) =

(i) $-CH_2-(CH_2)_n-$ or

(ii) $-(CH_2)_n-CO-$ or

(iii) $-CO-(CH_2)_n-$

where n = 0-3

$R^1$ and $R^2$, the same or different =

(i) alkyl (e.g. $^iPr$, $^iBu$, $^sBu$) or

(ii) $ArCH_2$ or

(iii) other lipophilic group (e.g. cyclohexyl-methyl) or

(iv) H (especially for $R^2$)

M = (i) $-CH(OH)-(CH_2)_n-$ or

(ii) $-CH(NH_2)-(CH_2)_n-$ or

(iii) $-CH_2-(CH_2)_n-$ or

(iv) $-CO-(CH_2)_n-$ or

(v) $-(CH_2)_n-N(R^7)-$, where $R^7$ = X

or

(vi) $-CH(NH_2)-(CH_2)_n-CO-NH-$

where n = 0-2

with the provisos I) that when M = (i), (iii) or (iv) and n = 1 then two three or four, and when M = (v) and n = 1 then three or four, of D, E, B and Z are present and preferably that when M = (i), (iii) or (iv) then two, three or four and when M = (v) then three or four of said residues are present

II) when M = (i), (ii) or (iv) then $R^5$ and $R^6$ are H and when M = (iii)

or (v) then if one of $R^4$, $R^5$, $R^6$ and $R^7$ is said group $-(CH_2)_n-OH$ or $-(CH_2)_n-NH_2$ the others, the same or different, are H or alkyl

(b) 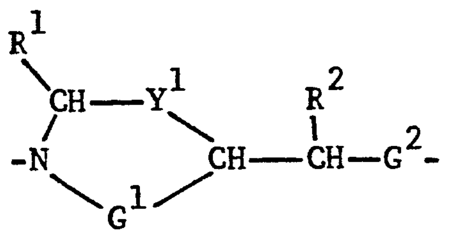 where

$R^1$, $R^2$ and $G^1$, $G^2$, the same or different, are as defined above and

$Y^1$ = (i) -CO- or

(ii) $-CH_2-$ or

(iii) -CH(OH)- or

(iv) $-CH(NH_2)-$ or

(v) $-CH_2-NR^3-$ ($R^3$ as above)

B = (a) absent or

(b) lipophilic or aromatic amino acyl (e.g. Val, Leu, Ile, Phe) either as such or $N^{\alpha}$-alkylated and/or reduced at carbonyl

Z = (a) absent or

(b) aromatic amino acyl (e.g. His, Phe, Tyr) or

(c) lipophilic amino acyl (e.g. cyclohexyl-alanyl), (b) and (c) being either as such or $N^{\alpha}$-alkylated and/or reduced at carbonyl ,

W = (a) -OH or other terminal group including those set out for W in general formula XV

 

(b) $-OR^3$ } $R^3$ as above

(c) $-NH_2$, $-NHR^3$, $-NR_2{}^3$ }

(d) 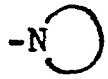 where N is part of a heterocyclic ring, preferably 5- or 6-membered, containing 1-3 heteroatoms (N, O or S) and of any degree of saturation and optionally substituted with $R^3$- or $R^3CH_2$-groups at one or more positions ($R^3$ as above)

<u>or</u>

Z-W = 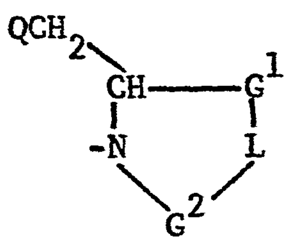

where L = (i) -NH- or

(ii) -O- or

(iii) $-NR^3-$

and $R^3$ and $G^1$ and $G^2$, the same or different, are as defined above and

Q = (i) H or

(ii) $C_{1-4}$ alkyl or

(iii) aryl or

(iv) imidazol-4-yl- or indol-3-yl

together with compounds in which, when one or more of the peptide bonds of the chain is represented by a 'reduced' isostere, the N atom of such isostere and the preceding or succeeding N atom in the chain are linked by a moiety as defined for $G^1$ and giving a five or six membered ring, and together further with compounds in which (except when M = (i), (iii), (iv) or (v), at least for n = 1) the above residues are present with further, N- or C- terminal,

aminoacyl or aminoacyl analogue residue(s), particularly Pro or J-Pro interposed between X and D, J being His or other basic or aromatic aminoacyl residue.

In the above, compounds where, in M, n = 1 are preferred. Of the isosteric links, the hydroxy ones are of particular value as giving high bonding affinity, from their close relation to the transition stage of the scissile (peptide) bond. Other important isosteric links are the 'amino', 'cyclised' and 'aminostatine' links where respectively A is a) & M = (ii) ; A is b); and A is a) & M =(vi) .

All the compounds may, further, be in the form shown or modified by replacement of one or more remaining peptide bonds by analogues M, e.g. reduced $-CH_2NH-$, keto $-CO-CH_2-$, hydroxy $-CH(OH)-CH_2$, amino $-CH(NH_2)-CH_2-$ or hydrocarbon $-CH_2-CH_2-$ isosteric linkages. They may be in the free form or in a protected form at one or more of the reactive functional groups such as amino, imino, carboxyl, hydroxyl etc., including peptide nitrogen. Furthermore the compounds may be present in the form of their physiologically acceptable acid addition salts or other devivatives convertible in the body to active form, which salts and derivatives are to be understood in the description and claims to be comprehended in the definitions of the compounds themselves.

Particular compounds of the present invention, showing desirable renin inhibitory action, are of the general formula:

X—Phe-His—A—B—Z—W (XV)

8 9 10,11 12 13

where Phe and His are optional (but are not both absent when A has the 'reduced' link below) and may further be in substituted form e.g. Phe by OH F Cl Br or Me, preferably at the 4-position, or His by Me; or Phe is replaced by Tyr, or His by spinacin

X = H; or an acyl or other N-protecting group, e.g. acetyl, pivaloyl, t-butyloxycarbonyl (Boc), benzoyl or lower alkyl (primarily $C_1$-$C_5$); or an L- or D- amino-acyl residue, which may itself be N-protected similarly and in particular may be Gly or D- or L- Pro, Val or Ile

A = 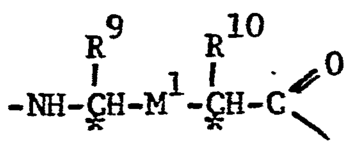 where $M^1$ is as M above or particularly a 'reduced' 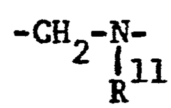 or 'keto' 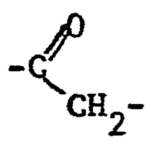 or 'hydroxy' -CH(OH)-$CH_2$-, or 'hydrocarbon' -$CH_2$-$CH_2$- isostere bond where the configuration at asymmetric centres is either R or S, where in the hydroxy isostere the hydroxy group may be present as such or protected in ether -$OR^{12}$ or ester 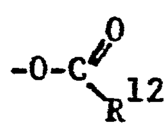 form where $R^{12}$

is as given under W below and where $R^9$ and $R^{10}$, the same or different = $^iPro$ (isopropyl), $^iBu$ (isobutyl), Bzl (benzyl) or other amino-acid side chain preferably lipophilic or aromatic;

$R^{11}$ = -H or an N-protecting group such as lower alkyl or lower acyl ($C_1$-$C_5$), or t-butyloxycarbonyl or benzyloxycarbonyl as such or ring substituted, or aryl sulphonyl e.g. $-SO_2Ph$ or $-SO_2-C_6H_4CH_3$(p), or formyl;

B = D- or L- Val Leu or Ile or other D- or L- lipophilic amino-acyl residue;

Z = D- or L- Tyr, Phe, His or other L- or D- aromatic amino-acyl residue; and

W = (i) -OH as such or in protected ester form as $-OR^{12}$ where $R^{12}$ = lower alkyl primarily $C_1$-$C_5$ and particularly $^tBu$, or cycloalkyl primarily $C_3$-$C_7$, or other ester forming group; or

(ii) $-NH_2$ as such or in protected amide form as $-NHR^{13}$ or $-N(R^{13})_2$ (where $R^{13}$ = an N-protecting or other substituent group e.g. lower alkyl as for $R^{12}$, and $(R^{13})_2$ = two such or e.g. cyclo-alkyl, primarily $C_3$-$C_7$) or as $-NH-(CH_2)_n-Q^1$ or $-NR^{13}-(CH_2)_n-Q^1$ (where n = 2 to 6 and $Q^1$ = $NH_2$ or

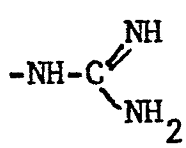 and wherein any of the hydrogens

attached to nitrogen may be substituted by $R^{13}$ or $(R^{13})_2$); or

(iii) an L- or D- serine or L- or D-lysine, arginine or other basic amino-acyl residue as such or in amide form, substituted amide form or ester form e.g. containing a group or groups as given for $R^{12}$ and $R^{13}$ above as the case may be; or

(iv) an amino alcohol residue derived therefrom as such or protected in ester or ether form e.g. containing a group as given for $R^{12}$ above;

or

Z + W = an alcohol derived from L- or D- Tyr, Phe, His or other L- or D- aromatic amino-acyl residue as such or protected in ester or ether form as above.

As with the previously set out general formulae the compounds may be in the above form or modified by isosteric replacement of one or more retaining peptide bonds e.g. by reduced $-CH_2-NH-$, keto $-C(=O)-CH_2-$, hydroxy $-CH(OH)-CH_2-$, hydrocarbon $-CH_2-CH_2-$ or other analogue links M and further being in free form or in protected form at one or more remaining amino or amide (including peptide) nitrogen, carboxyl, hydroxy or other reactive groups, or in salt form at amino imidazole or carboxyl groups in particular as their physiologically acceptable acid addition salts at basic centres.

Protective or substituent groupings as mentioned above may be any of these known in the polypeptide art, amply disclosed in the literature and not requiring

discussion at length here. Generally the selection of the groups is according to their function, some being primarily intended to protect against undesired reaction during synthetic procedures while the N- and C- terminal substituents are for example directed against the attack of exopeptidases on the final compounds or to increase their solubility and hence physiological acceptability. All these functions are generally within the term "protective group" or the like used in the description and claims herein.

The invention further lies

(a) in a diagnostic test aimed at establishing the significance of renin as a causative or contributing factor in hypertension or hyperaldosteronism, and a surgical prognostic test for renovascular hypertension, in which there is administered a renin-inhibiting analogue according to the invention and blood pressure is monitored, and

(b) in the treatment of various forms of hypertension, particularly those forms associated with increased activity of the renin-angiotensin system, in which there is administered a renin inhibiting analogue according to the invention.

The long and short term response of blood pressure to renin inhibitors is predictive of surgical outcome. In all cases single and repeated doses and any conventional form of pharmaceutical composition may be used, for administration by intranasal or oral route, injection, or any other means as convenient. Amounts may for example be 0.001 to 10 mg/kg body weight

(calculated as free base) daily more usually 0.01 to 1 mg, according to the potency of the analogue and the severity of the condition. Dosage unit compositions may contain such amounts or submultiples thereof to make up the daily dose.

Examples of compounds according to the invention, with their activity and synthesis, follow. Individual synthesis schemes have numbering of the compounds not necessarily related to that of other schemes.

SECTION 'A' EXAMPLES 1 to 17 - LIST

Table 2 below is of Examples of compounds with the modified 10,11 isosteric links disclosed in European patent application A 0 045 665 but now applied to hexapeptide or smaller analogues.

## Table 2

| Ex. | Code | X | 8 | 9 | 10 | 11 | 12 | 13 | W | $IC_{50}$ µM v/s human renin |
|---|---|---|---|---|---|---|---|---|---|---|
| | | **Residue** | | | | | | | | |
| 1 | H184 | H- | | | Leu$\overset{R}{—}$ | Val | Ile | His | D-Lys-OH | |
| 2 | H185 | H- | | His | Leu$\overset{R}{—}$ | Val | Ile | His | D-Lys-OH | |
| 3 | H262 | H- | | | Leu$\overset{OH}{—}$ | Val | Ile | His | Lys-OH | 11 |
| 4 | H265 | Boc- | | | Leu$\overset{OH}{—}$ | Val | Ile | His-OH | | |
| 5 | H266 | H- | | | Leu$\overset{OH}{—}$ | Val | Ile | His-OH | | 29 |
| 6 | H267 | Boc- | | His | Leu$\overset{OH}{—}$ | Val | Ile | His-OH | | |
| 7 | H268 | H- | | His | Leu$\overset{OH}{—}$ | Val | Ile | His-OH | | |
| 8 | H269 | Boc- | Phe | His | Leu$\overset{OH}{—}$ | Val | Ile | His-OH | | 0.007 |
| 9 | H270 | H- | Phe | His | Leu$\overset{OH}{—}$ | Val | Ile | His-OH | | 5 |
| 10 | H282 | Boc- | Phe | His | Leu$\overset{OH}{—}$ | Gly | Ile | His-OH | | 0.3 |
| 11 | H286 | Boc- | Phe | His | Leu$\overset{OH}{—}$ | Val | Ile | Phe-OMe | | 0.018 |
| 12 | H287 | Boc- | Phe | His | Leu$\overset{OH}{—}$ | Val | Ile | | -OMe | 0.005 |
| 13 | H288 | Boc- | Phe | His | Leu$\overset{OH}{—}$ | Val | Ile | His-OMe | | 0.0035 |
| 14 | H289 | Boc- | Phe | His | Leu$\overset{K}{—}$ | Val | Ile | His-OH | | |
| 15 | H290 | Boc- | Phe | Ala | Leu$\overset{OH}{—}$ | Val | Ile | His-OH | | |
| 16 | H291 | Boc- | Phe | Phe | Leu$\overset{OH}{—}$ | Val | Ile | His-OH | | |
| 17 | H294 | Boc- | Phe | His | Leu$\overset{R}{—}$ | Val | Ile | His-OH | | |

SECTION 'A' cont'd - DIPEPTIDE ANALOGUE SYNTHESES

The synthesis of the reduced, keto, and hydroxy analogues in the above table is generally by the methods of European patent application A 0 045 665, the dipeptide analogues being prepared first and incorporated in the full sequence by essentially standard methods of peptide synthesis. Thus for example a hydroxy or keto isostere of a dipeptide may be made by a method wherein a derivative of a halohydrin, preferably a bromohydrin, or haloketone, preferably a bromoketone

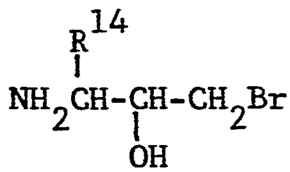

or

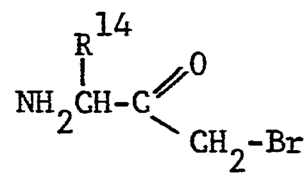

wherein $R^{14}$ is an amino acid side chain and the $NH_2$ and OH groups are in protected form, is subjected to an alkylation procedure to attach a group 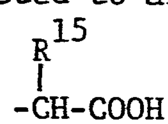 giving the desired isostere as such or in protected form, $R^{15}$ being the same or a different amino acid side chain.

In particular the alkylation procedure may be

i) by reaction with an alkali metal carboxylic acid derivative preferably a lithium derivative 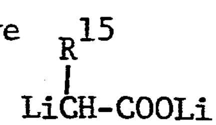 where $R^{15}$ is as above.

ii) by reaction with an alkali metal malonic ester derivative preferably a sodium derivative 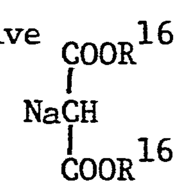

where $R^{16}$ is an esterifying group and a halide preferably an iodide, $R^{15}$-I, where $R^{15}$ is as above, to give the intermediate:

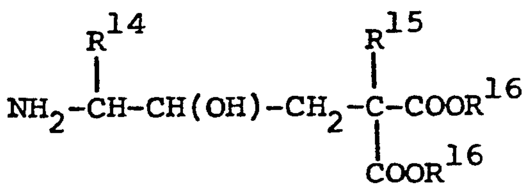

or

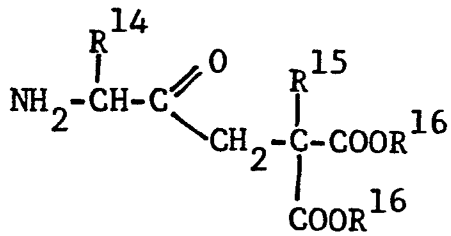

in protected form which intermediate is then decarboxylated and if desired deprotected to give the desired isostere:

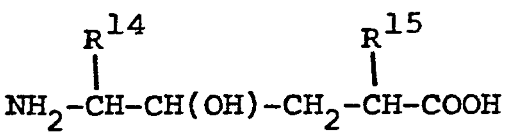

or

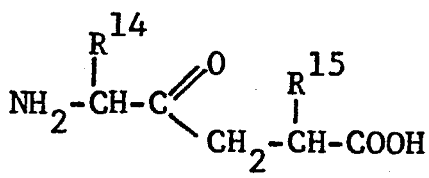

as such or in protected form.

The hydroxy isosteres so produced may further be oxidised to the corresponding keto isosteres.

A further, preferred, synthesis of hydroxy and hence keto dipeptide isosteres is given below, applied to Leu-hydroxy-Val and referring to Scheme 1 following.

Synthesis of Protected Leu-OH-Val 1a

(1) Phthalimido-ketol 42

(a) A solution of phthaloyl-L-leucine (60mmol) in ethyl acetate (150ml) was cooled to -10°C, N-methyl-morpholine (60mmol) was added followed by iso-butyl chloroformate (60mmol) at such a rate that the temperature never exceeded -10°C. After 10 mins. at -10°C, the N-methyl-morpholine hydrochloride was filtered off and the solution of mixed anhydride poured into a solution of diazomethane (approx. 130mmol, dried over KOH pellets) in ether (500ml). After 3 hours at 22°C, the solvent was evaporated and the crude diazoketone 41 was obtained as an orange oil. IR Spectrum (film) $\nu$max 2100 $cm^{-1}$.

(b) The above diazoketone was dissolved in dioxan (200ml), 1M $H_2SO_4$ (100ml) added and the mixture was heated at 75° for 30 mins. The reaction mixture was cooled, its pH adjusted by the slow addition of $NaHCO_3$ to 5 and the dioxan was evaporated. The remaining aqueous solution was extracted with ethyl acetate, the extracts were washed with water and brine, dried over $Na_2SO_4$ and evaporated. The crude ketol 42 was crystallised from ether-petrol to give pale yellow platelets. In a subsequent preparation the crude ketol was found to be satisfactory by TLC (in petrol (60-80°) containing 30% ethyl acetate) and was used without crystallisation.

(2) tert-Butyl ether 43

The ketol 42 from (1) (b) (50mmol) was dissolved in dichloromethane (50ml), cooled to -78°C and liquid iso-butene (50ml) and concentrated sulphuric acid (0.5ml) were added. The reaction mixture was stoppered and kept at 22°C for 72 hrs.

The reaction vessel was cooled and opened, and the pH of its contents was adjusted to 5 with $NaHCO_3$. The product was extracted with ether; ethereal extracts were washed with water, brine, dried over $Na_2SO_4$ and evaporated to yield the tert-butyl ether (98%).

(3) Diols 44a and 44b

A solution of the tert-butyl ether 43 (50mmol) in tetrahydrofuran (150ml) was cooled to $0^oC$. To it were added 1M HCl (25ml) followed over a period of 20 mins. by $NaCNBH_3$ (250mmol) in a mixture of tetrahydrofuran (100ml) and water (20ml). After stirring for 2 hrs. the pH of the solution was adjusted to 4, tetrahydrofuran was evaporated in vacuo, the residue was triturated with ether, the ethereal solution was washed with water and brine, dried and evaporated. A 3:1 mixture of the two diastereomers was obtained in 90% yield, the major component being the desired 2R,3L compound 44a. Progress of the reduction was monitored by TLC in chloroform.

(4) Benzyl ethers 45a and 45b

(a) A mixture of the diastereomeric alcohols from (3) (40mmol) was azeotroped three times with dry benzene and then dissolved in dry dimethyl formamide (100ml). Under dry nitrogen, NaH (1 equivalent) was added with cooling in ice-water. After all NaH had reacted (approx. 10 mins.), benzyl bromide (1.5 equivalents) was added. The reaction mixture was stirred at $20^oC$ and the progress of benzylation was monitored by TLC in chloroform: petrol ($60-80^o$) = 4:1 or in petrol ($60-80^o$) containing 10% ethyl acetate. It was complete after 2.5 hrs., when the reaction mixture was cooled, acidified with 1M citric acid and evaporated in vacuo. The residue was taken up in ether, washed with water and brine, dried and evaporated.

(b) Separation of the diastereomers 46a and 46b. The mixture from (a) was adsorbed onto silica, dried in vacuo and added to the top of a dry-packed column, which was eluted with petrol (60-80°) containing 5% ethyl acetate. The major diastereomer 45a was eluted from the column first. Configuration at C-2 and C-3 was determined by X-ray crystallography and was found to be 2R, 3L for the major diastereomer 45a.

(5) Mesylate 47a

(a) A solution of the major benzyl ether 45a (20mmol) in dichloromethane (50ml) was treated in an atmosphere of nitrogen with trifluoroacetic acid (50ml). After 1.5 hrs. the reaction mixture was evaporated to dryness, and the residue taken up in ethyl acetate. The solution was carefully washed to neutrality with 1M $NaHCO_3$, water and brine, dried and evaporated to give the alcohol 46a. The latter can be crystallised from ethyl acetate-petrol (60-80°) to furnish colorless needles, but chromatography on silica may be necessary to obtain a higher yield.

(b) A solution of the alcohol 46a from (a) (20mmol) in dichloromethane (100ml) was cooled to 0°, triethylamine (2 equivalents) and methane-sulphonyl chloride (1.1 equivalents) were added and the reaction mixture was stirred at 22° for 1 hr. It was evaporated, the residue dissolved in ethyl acetate, this solution washed with water and brine, dried and evaporated to leave an oil which crystallised on standing. The latter was recrystallised from dichloromethane-petrol (60-80°) to give the mesylate 47a as glistening plates (90%).

(6) Malonic ester 48a

Di-tert-butyl malonate (24mmol) was added to a slurry of sodium hydride (24mmol) in 1,2-dimethoxyethane (120ml) under dry nitrogen. After 10 mins., crystalline mesylate 47a (20mmol) was

added and the mixture was refluxed under nitrogen. Progress of the alkylation was monitored by TLC in chloroform-petrol (60-80°) (7:3), the $R_F$ of the product being slightly higher than that of the mesylate. Reaction was complete after 24 hrs, when the mixture was cooled and poured into 1M citric acid solution. Solvents were evaporated in vacuo, the residue taken up in ethyl acetate, washed with water and brine, dried and evaporated to yield crude malonic ester 48a. The latter was purified by chromatography on a dry column of silica as described above for 45a in section (4) (b). Elution was carried out with chloroform-petrol (3:2) to give pure 48a in 67% yield as a colorless oil.

(7) iso-Propyl malonic ester 49a

Malonic ester 48a (15mmol) was azeotroped three times with dry benzene, dissolved in dry tetrahydrofuran (50ml) and added to a slurry of sodium hydride (1.1 equivalents) in tetrahydrofuran under dry nitrogen. After refluxing for 20 mins., isopropyl iodide (10 equivalents) was added and the mixture refluxed for a further hour. After cooling, 1M citric acid solution was added, the solvent evaporated and the residue taken up in ethyl acetate. After washing with water and brine and drying, ether was evaporated to leave the iso-propyl derivative 49a as a colorless oil (67%). Purity was checked by TLC in chloroform-petrol (7:3), and the product was either used directly in step (8) or, if necessary, first purified by chromatography on silica in chloroform-petrol (60-80°) (3:2).

(8) Phthaloylamino carboxylic acids 50a $\alpha$ and $\beta$ .

The di-tert-butyl ester 49a (15mmol) was treated with 50% trifluroroacetic acid in dichloromethane (100ml) under nitrogen for 1.5 hrs. to remove the tert-butyl ester groups.

This mixture was evaporated to dryness, the residue dissolved in toluene and refluxed for 6 hrs. Decarboxylation was monitored by TLC in chloroform-methanol-acetic acid (62:4:1). On completion, the reaction mixture was evaporated to dryness, the last traces of solvent being removed by pumping in high vacuum. A mixture of the epimeric acids 50aα and 50aβ was obtained as an oil, and was either used directly in step (9) or, if necessary, purified by chromatography on silica using chloroform-methanol-acetic acid (97:2:1) for elution.

(9) Amino carboxylic acids 51a α and β

The phthaloyl derivatives 50aα and β (10mmol) were dissolved in ethanol (80ml) and treated with hydrazine hydrate (10 equivalents) under reflux for 1.5 hrs. Removal of the phthaloyl group was monitored by TLC in chloroform-methanol-acetic acid (62:4:1). On completion, the reaction mixture was cooled, water was added to dissolve the precipitate formed, solvents were removed in vacuo and the residue was dried in vacuo over concentrated $H_2SO_4$ overnight. It was dissolved in the minimum amount of water, extracted with ether (10ml), acetic acid was added to pH = 4, the mixture cooled and the precipitated phthaloyl hydrazide was filtered off. Evaporation of the filtrate yielded a mixture of the amino acids 51aα and β.

(10) Boc-Amino acid 1a

(a) Excess $KHCO_3$ was added to a solution of the amino acids 51a in water (20ml), followed by di-tert-butyl dicarbonate (excess, depending on the amount of residual hydrazine present in 51a) dissolved in dioxan (20ml). On completion of the reaction the pH was adjusted to 4, solvents were evaporated, the residue was dissolved in ethyl acetate and the solution was washed with water and brine.

After drying, the solvent was removed in vacuo, the residue triturated with petrol (60-80°) and any N, N'-bis-Boc-hydrazine present removed by filtration. Evaporation of the petrol yielded crude Boc-acids 52a α and β. These were dissolved in ethyl acetate, N,N-dimethylaminoethylamine (approx. 2g) was added to react with the excess di-tert-butyl dicarbonate present, and after 20 mins. the solution was extracted with 1M citric acid. It was washed with water, brine, dried and evaporated to give 52a α and β as a pale yellow oil.

(b) The above mixture of epimeric acids was separated by chromatography on silica gel (40-60μ, dry-packed column) eluting either with petrol containing 20% ethyl acetate (and possibly a small amount of acetic acid to prevent trailing), or with chloroform containing 2% methanol. Separation was monitored by TLC in chloroform-methanol-acetic acid (97:2:1). The more polar component was the required 2L, 4S, 5L epimer 1a.

Scheme I

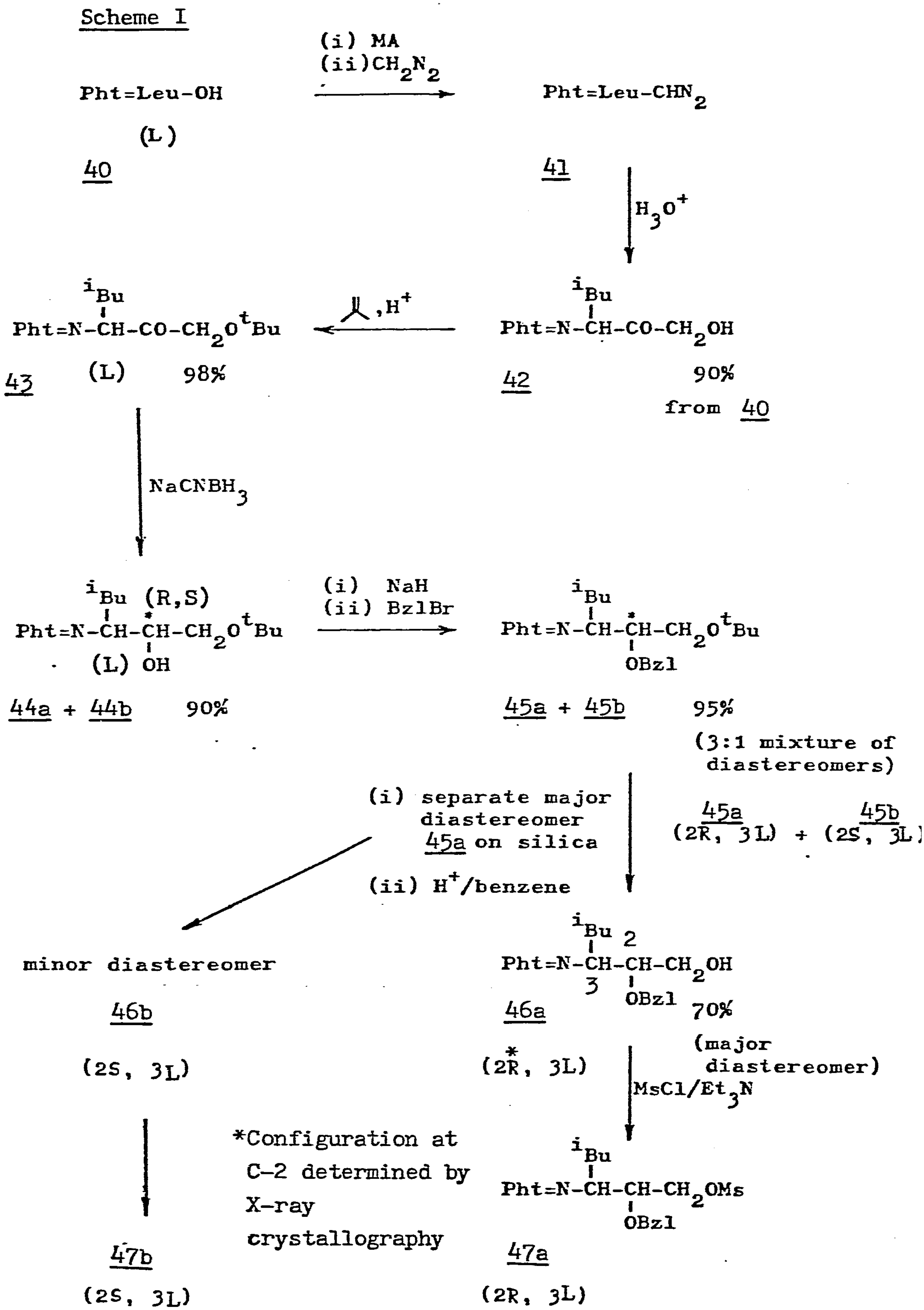
(i) MA
(ii) $CH_2N_2$
(L)
40
41
$H_3O^+$
, $H^+$
43
(L)
98%
42
90%
from 40
$NaCNBH_3$
(R,S)
(L)
44a + 44b
90%
(i) NaH
(ii) BzlBr
45a + 45b
95%
(3:1 mixture of diastereomers)
45a (2R, 3L) + 45b (2S, 3L)
(i) separate major diastereomer 45a on silica
(ii) $H^+$/benzene
minor diastereomer
46b
(2S, 3L)
46a
70%
(major diastereomer)
(2R*, 3L)
$MsCl/Et_3N$
*Configuration at C-2 determined by X-ray crystallography
47b
(2S, 3L)
47a
(2R, 3L)

Scheme I (cont'd)

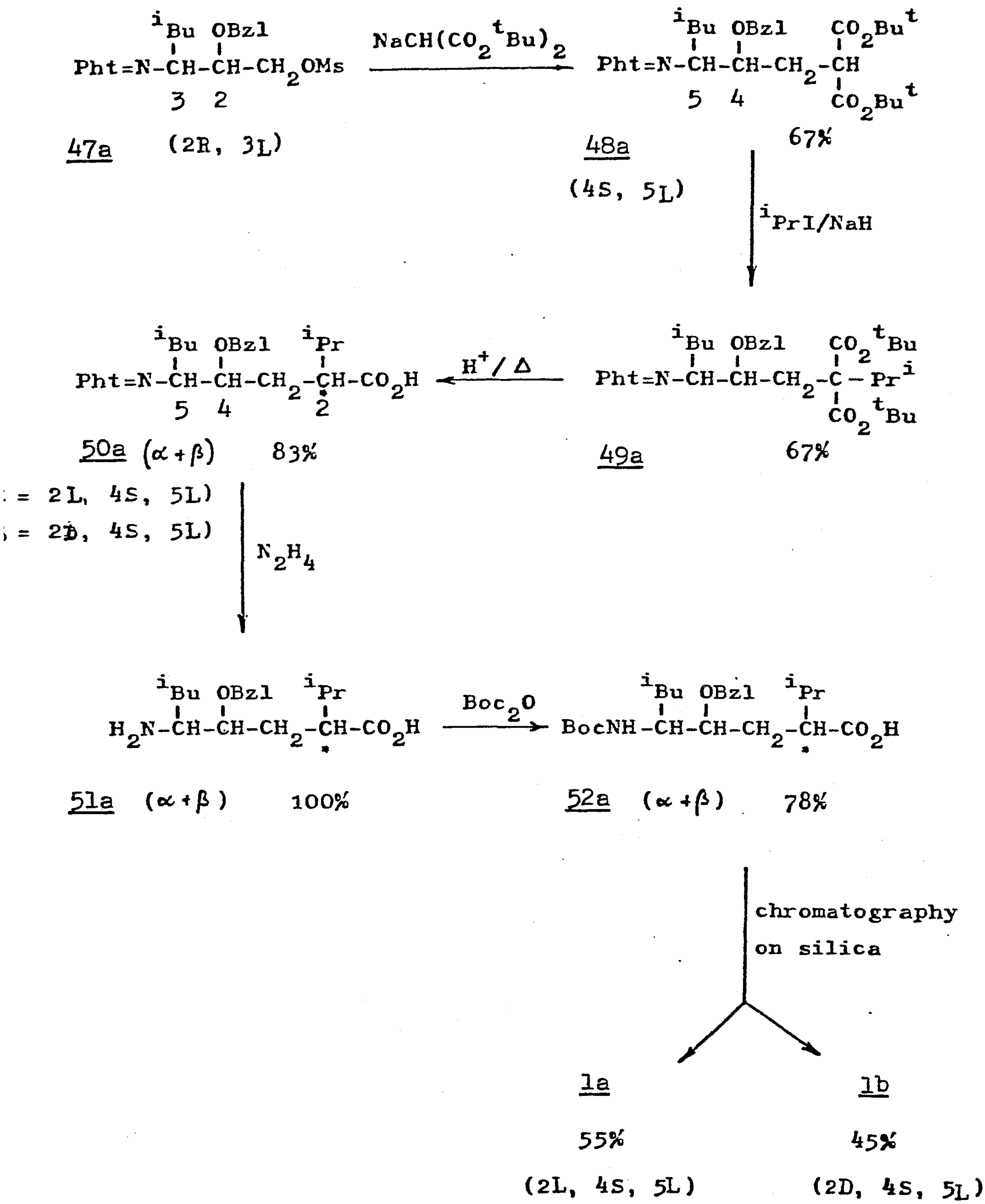

## SECTION 'A' cont'd - SYNTHESIS OF ACTIVE COMPOUNDS

### H184, H185 (Examples 1, 2)

Starting from the methyl ester of Boc-L-leucine a protected Leu-reduced-Val isostere is made by the synthetic route set out in Scheme 3, pages 16 to 18, of European Patent Specification A 0 045 665. It is converted to Leu-reduced-Val Ile His D-Lys-OH, His Leu-reduced-Val Ile His D-Lys-OH and Phe His Leu-reduced-Val Ile His D-Lys-OH by standard methods of peptide synthesis, as illustrated in that specification.

### H262 (Example 3)

The Leu-OH-Val dipeptide isostere is prepared as given above and converted to Leu-OH-Val Ile His Lys-OH by, again, standard methods.

### H265 and H266, H267 and H268, H269 and H270 (Examples 4 - 9)

These compounds, three pairs of respectively N-Boc protected and free N-terminal $NH_2$ isosteres, all with C-terminal Ile His-OH, are made starting with the protected Leu-OH-Val isostere prepared as given above. The synthesis otherwise is by known methods,

being in substance as in Scheme 2 below showing the synthesis of compounds, not themselves within the present invention, of structure (two forms):

H-His Pro Phe His Leu-OH-Val Ile His-OH

(H194, H195)

Scheme 2

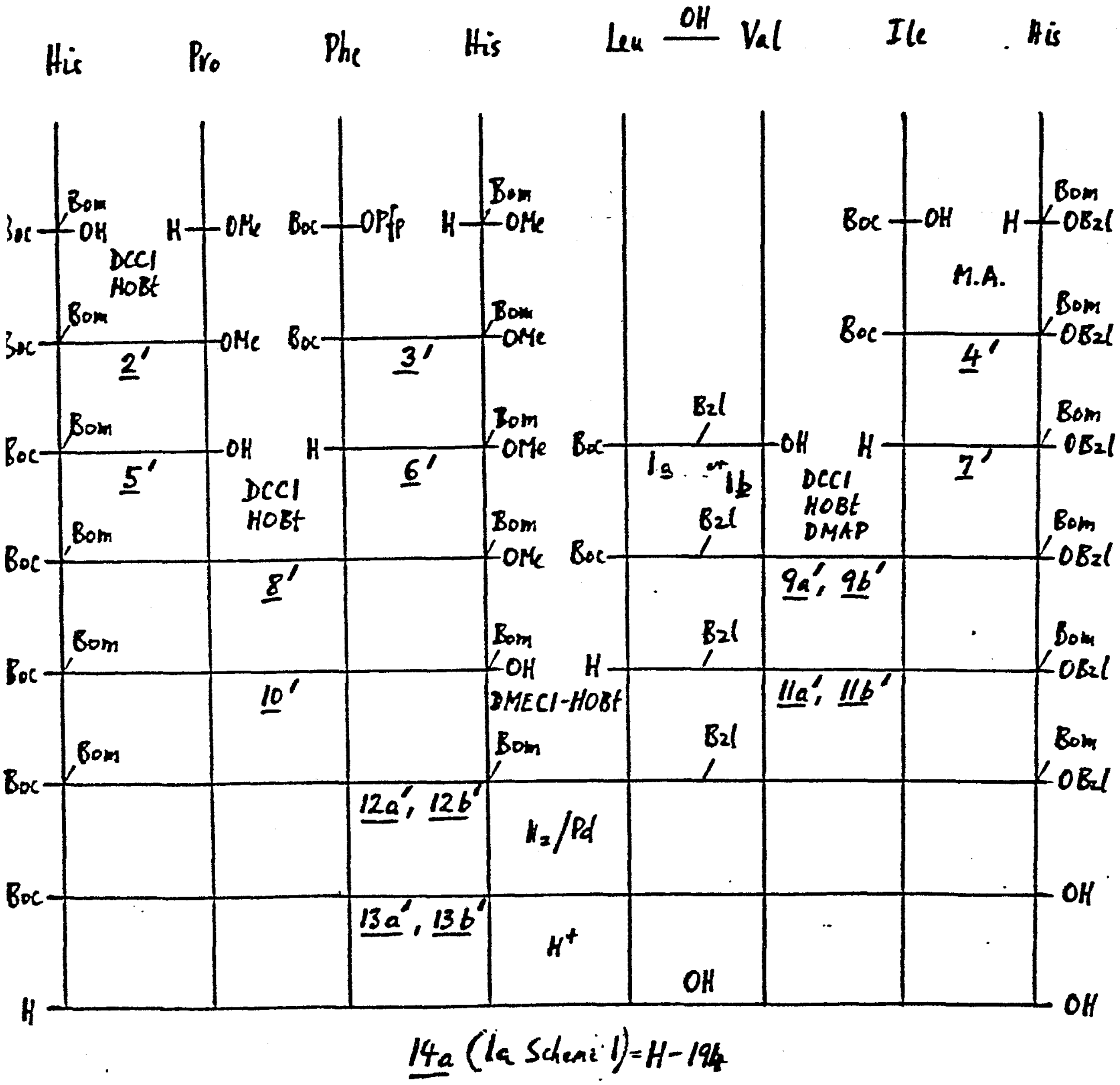


14a (Ia Scheme I)=H-194

14b (Ib Scheme I)=H-195

13a' = H-261

H282 (Example 10)

This analogue contains Leu-OH-Gly, which is synthesised according to Scheme 1 except that the alkylation step with isopropyl iodide producing 49a from 48a is omitted, the synthesis proceeding on 48a. Otherwise the synthesis is as for H269.

H286, H287, H288 (Examples 11 - 13)

These compounds, containing Leu-OH-Val isosteres, are made essentially as H269.

H289 (Example 14)

The protected Leu-OH-Val isostere made according to Scheme I herein is converted to the corresponding Leu-keto-Val isostere as follows:

Scheme 3

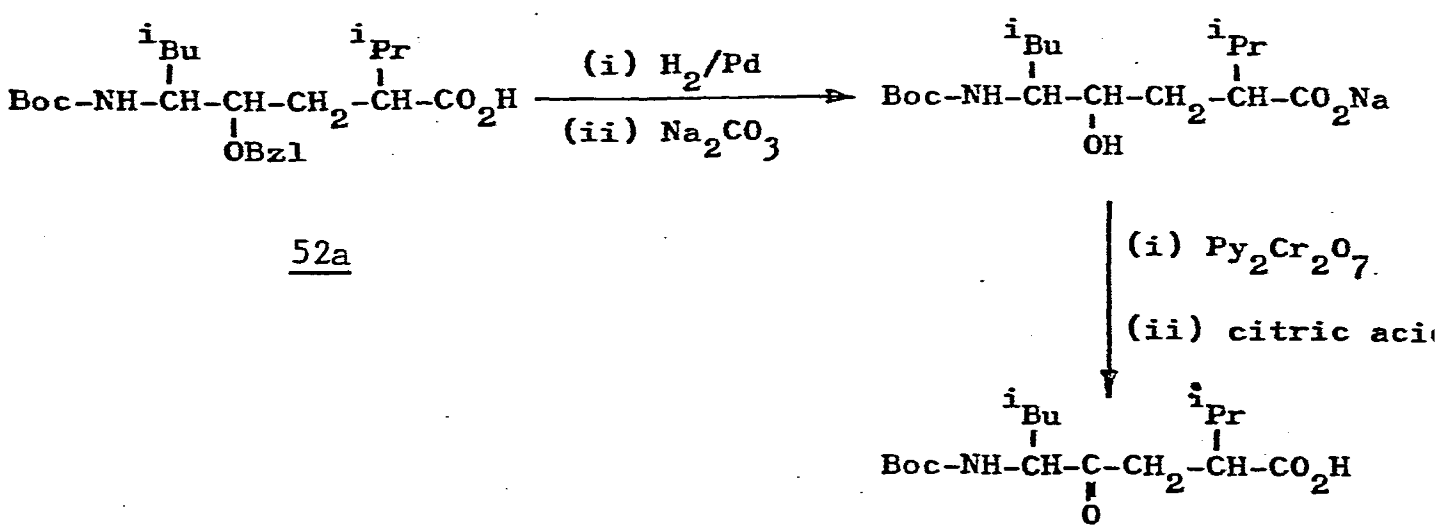


52a

The keto isostere is then used in a synthesis like that of H269.

H290, H291 (Examples 15, 16) and H294 (Example 17)

These syntheses are that of H269 modified to couple Ala or Phe to the Leu of the dipeptide isostere residue instead of His,or in the case of H294 simply using the Leu-R-Val isostere of Examples 1 and 2 in place of the hydroxy isostere in a synthesis otherwise as that of H269.

SECTION 'B' EXAMPLES 18 to 28 - LIST

The following examples are of renin-inhibiting peptide analogues containing isosteric links different from those of European patent specification A 0 045 665, in the form of a table of structures followed by details of syntheses.

Table 3

| Ex. | Structures |
|---|---|
| 18 | Boc-Phe His Leu$\overset{NH_2}{\text{——}}$Val Ile His-$NH_2$ |
| | 8 9 10 11 12 13 |
| 19 | Boc-Phe His Leu$\overset{NH_2}{\text{——}}$Val Ile-NH-$CH_2CH_2$Ph |
| | 8 9 10 11 12 13 |
| 20 | Boc-Phe$\overset{Me}{\text{——}}$Phe Leu$\overset{NH_2}{\text{——}}$Val Ile-NH-$CH_2CH_2$-(2-pyridyl) |
| | 8 9 10 11 12 13 |
| 21 | Boc-Phe His Ast$^{(R)}$ Ile His-OH |
| | 8 9 10 11 13 |

Table 3 (cont'd)

| Ex. | Structures |
|---|---|
| 22 | Boc-Phe His Ast$^{(S)}$ Ile His-OH<br>8 9 10 11 13 |
| 23 | Boc-Phe His-N-CH($^i$Bu)-CH$_2$-NH-CH($^i$Pr)-CO-Ile His-OH<br>CH$_2$CH$_2$NH$_2$<br>8 9 10 11 12 13 |
| 24 | 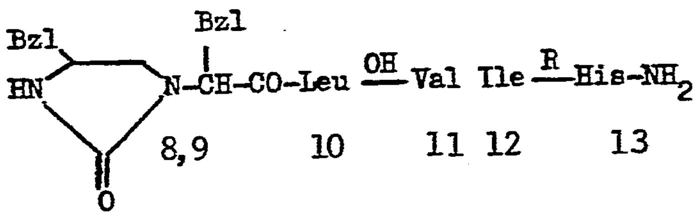<br>8,9 10 11 12 13 |
| 25 | 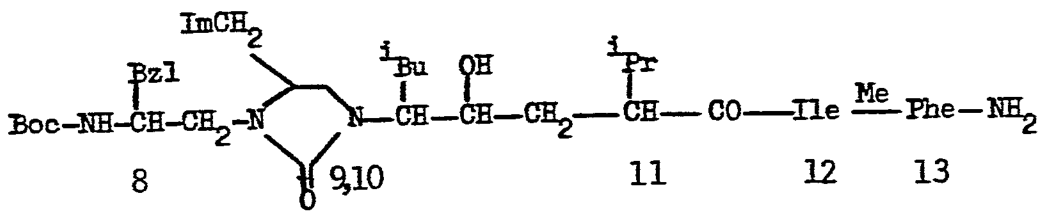<br>8 9,10 11 12 13 |
| 26 | 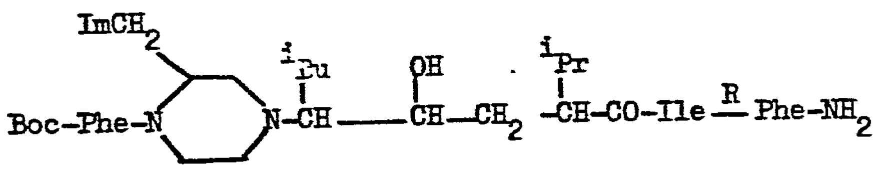<br>8 9,10 11 12 13 |
| 27 | 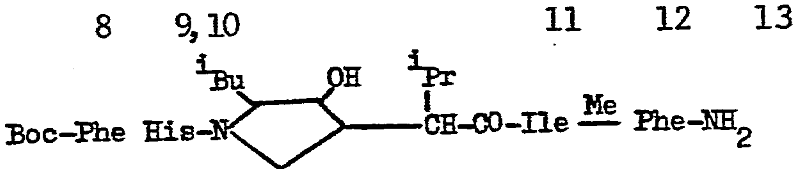<br>8 9 10,11 12 13 |
| 28 | 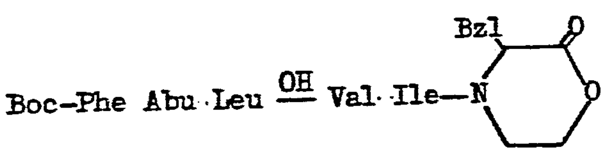<br>8 9 10 11 12 13 |

SECTION 'B' cont'd - SYNTHETIC METHODS

A synthesis route to the novel amino isostere $-CH(NH_2)-CH_2-$ is illustrated in Scheme 4 below which shows the preparation of the protected Leu$\overset{NH_2}{—}$Val isostere 17. The latter can be incorporated into peptides, e.g. into the compounds of Examples 18 to 20 by standard methods of peptide synthesis as for example already described in the European Patent Application 0 045 665 and referred to herein. Correspondingly, Scheme 5 shows the synthesis of protected 3-amino-3-deoxy-statine, and in the analogues of Examples 21, 22.

The compound of Example 23 is essentially a 'reduced' isostere of the kind described and synthesised in European Application 0 045 665 but with an amino-ethyl substituent on the peptide nitrogen of residue 10.

The syntheses of the novel cyclic structures representing one or two residues in the backbone and present in the compounds of Examples 24-28 are shown in Schemes 6-10. Again, incorporation into the final sequence is essentially by standard methods. These cyclic structures serve to stabilize the enzyme-bound conformation of each transition-state analogue, and to render the peptide backbone more resistant to attack by peptidases.

Scheme 4 (Examples 18 - 20)

Synthesis of protected amino isostere 17

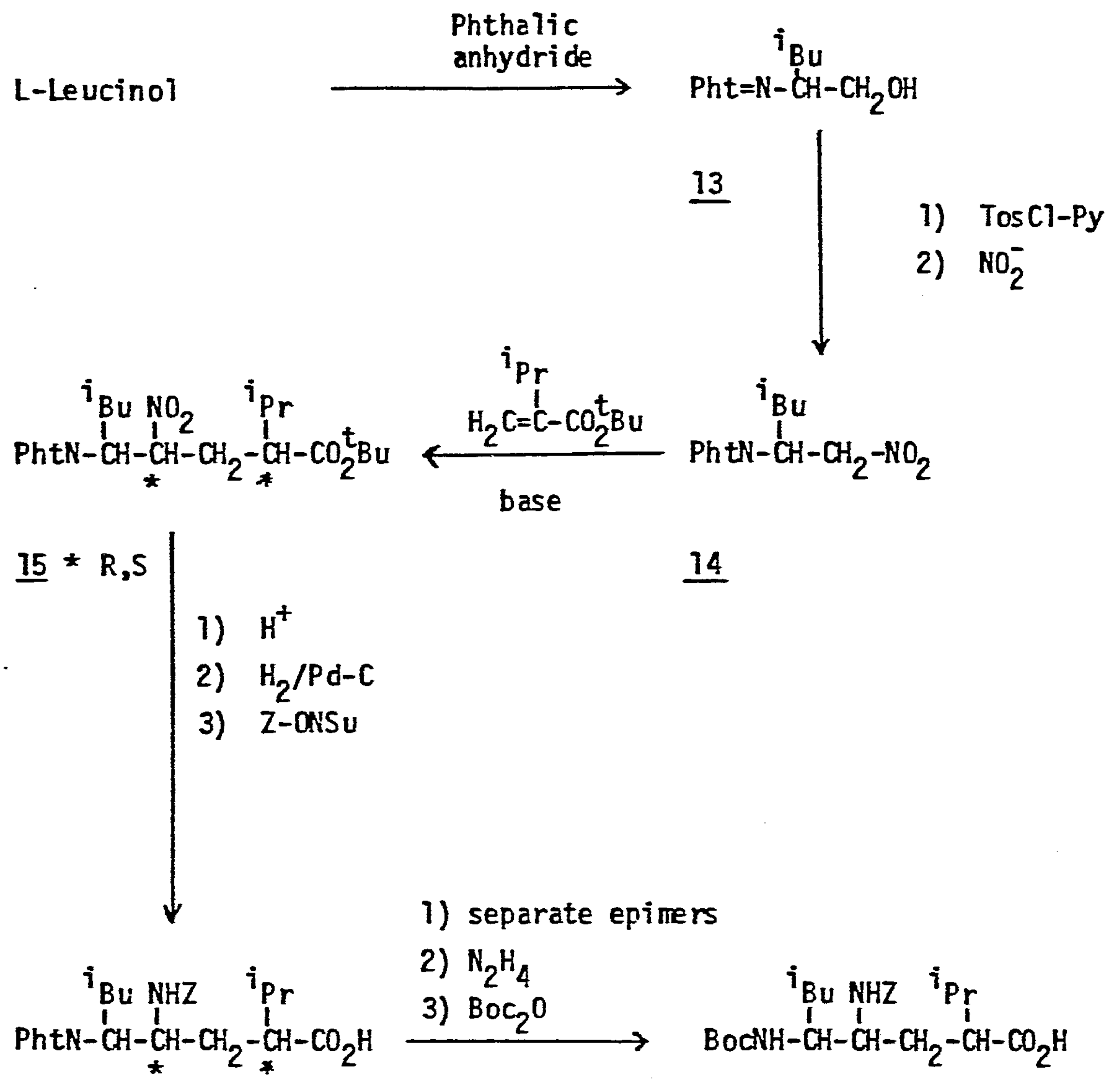


16 17

As noted above the isostere 17 is incorporated into the full sequence of Examples 18 to 20 by the standard methods of peptide synthesis, with the C-terminal free in the compound of Example 18 (His) and protected by $-CH_2CH_2Ph$ and $-CH_2CH_2$-(2-pyridyl) in Examples 19 and 20 (Ile). In the C-terminal sequence Boc-Phe$\overset{Me}{—}$Phe- (Example 20), the methyl is on the peptide nitrogen of the Phe-Phe link.

Scheme 5 (Examples 21, 22)

(A) Synthesis of protected 3-amino 3-deoxy -statine 39

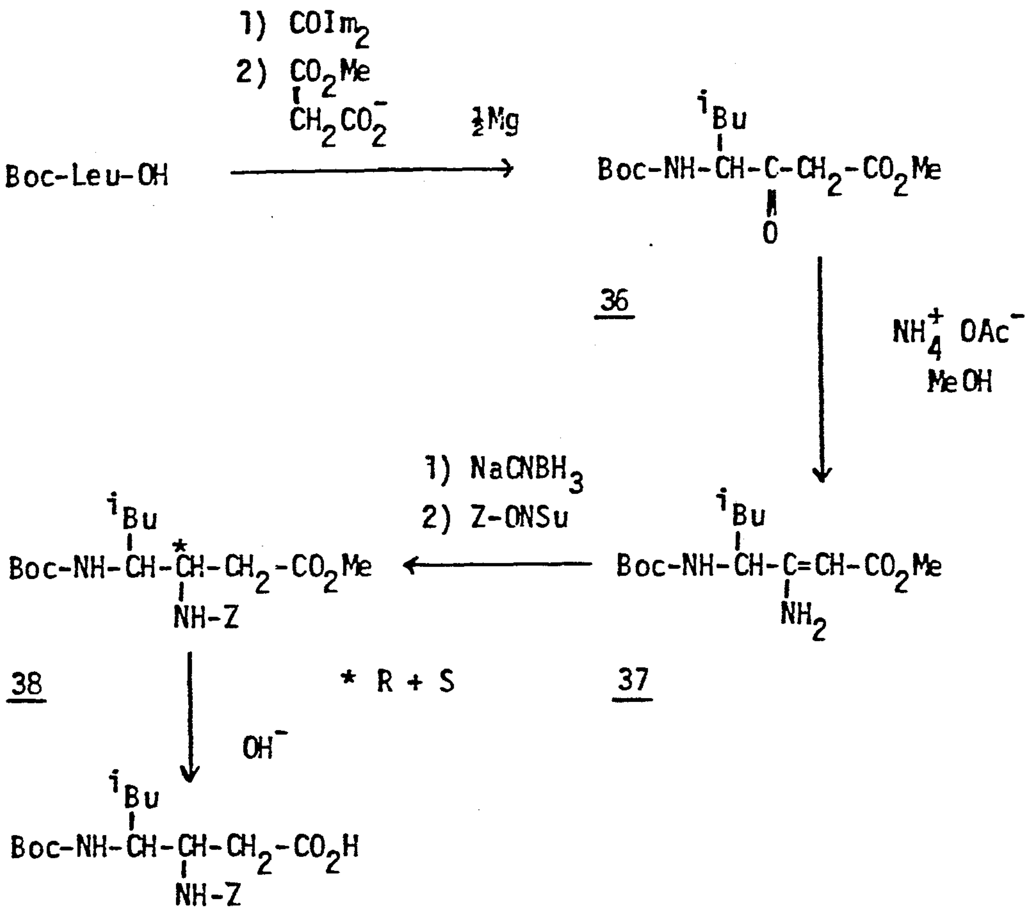


36

37

38

39

(B) Incorporation of 3-amino-3-deoxy-statine 39 into H-292 and 293.

39 is first coupled via DCCI/HOBt to H-Ile-His(Bom)-OBzl, deprotected at the N-terminus with HCl-dioxan and then extended by stepwise coupling of Boc-His(Bom)-OH and Boc-Phe-OH. The protected peptide is separated into the two epimers differing in configuration at the carbon atom bearing the amino substituent, and each one is deprotected by $H_2$/Pd-C in the presence of semicarbazide to give, respectively, H-292 (Example 21) and 293 (Example 22).

Scheme 6 (Example 24)

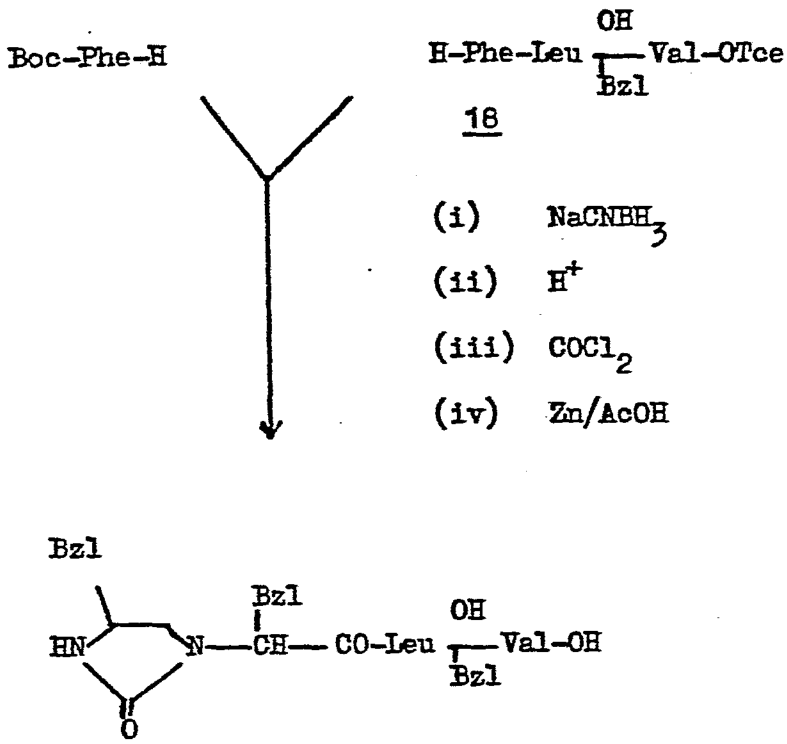


18

19

The analogue 18 is first made by the methods described herein, then converted to the cyclic form, as shown, before reaction with an Ile$\overset{R}{-}$His dipeptide analogue to give the analogue of Example 24.

Scheme 7 (Example 25)

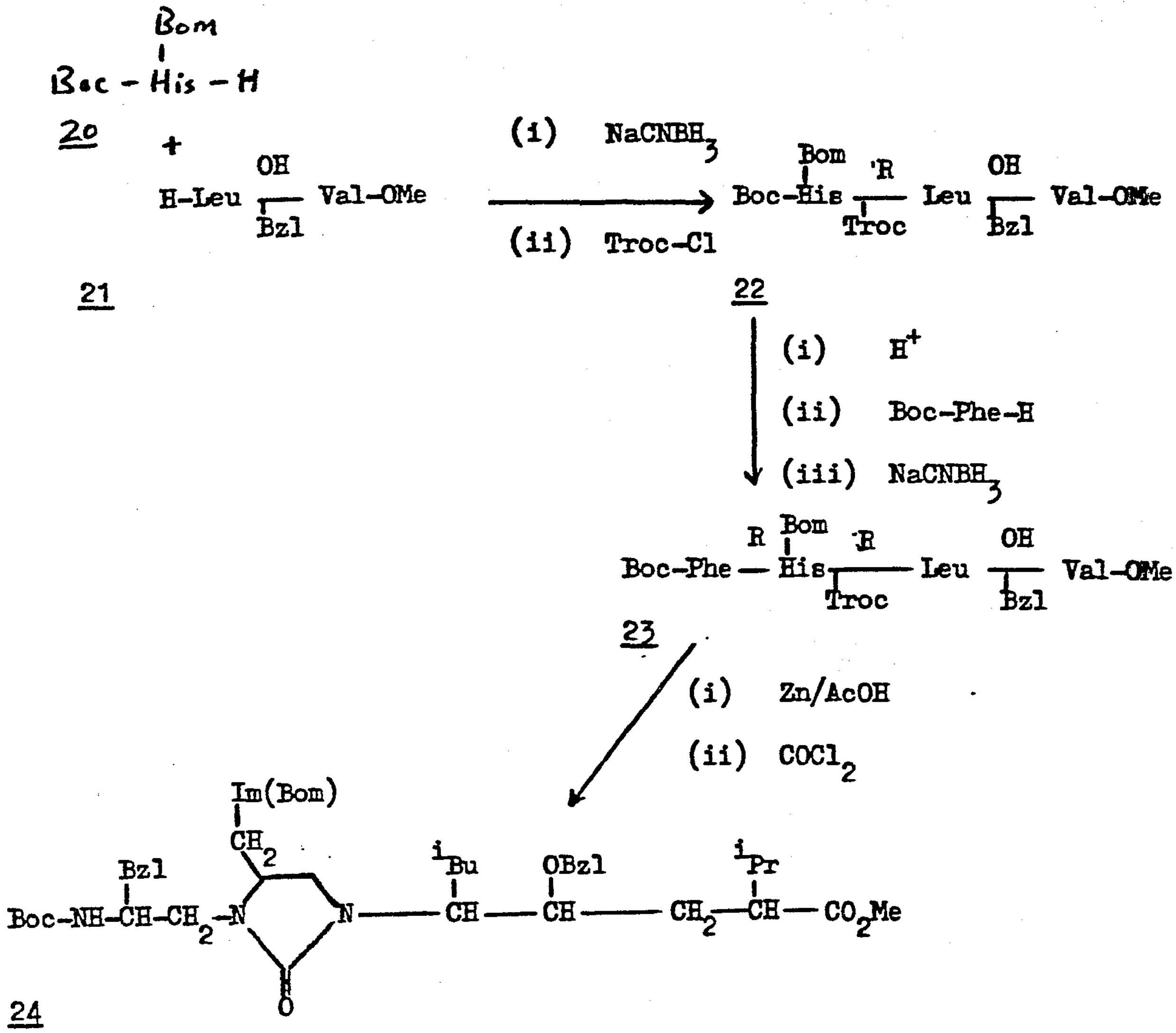


20 21 22 23 24

The synthesis above is followed to give compound 24 then the final amide protected residue Ile$\overset{Me}{—}$Phe-$NH_2$ attached, deprotecting as necessary to give the analogue of Example 25.

Scheme 8 (Example 26)

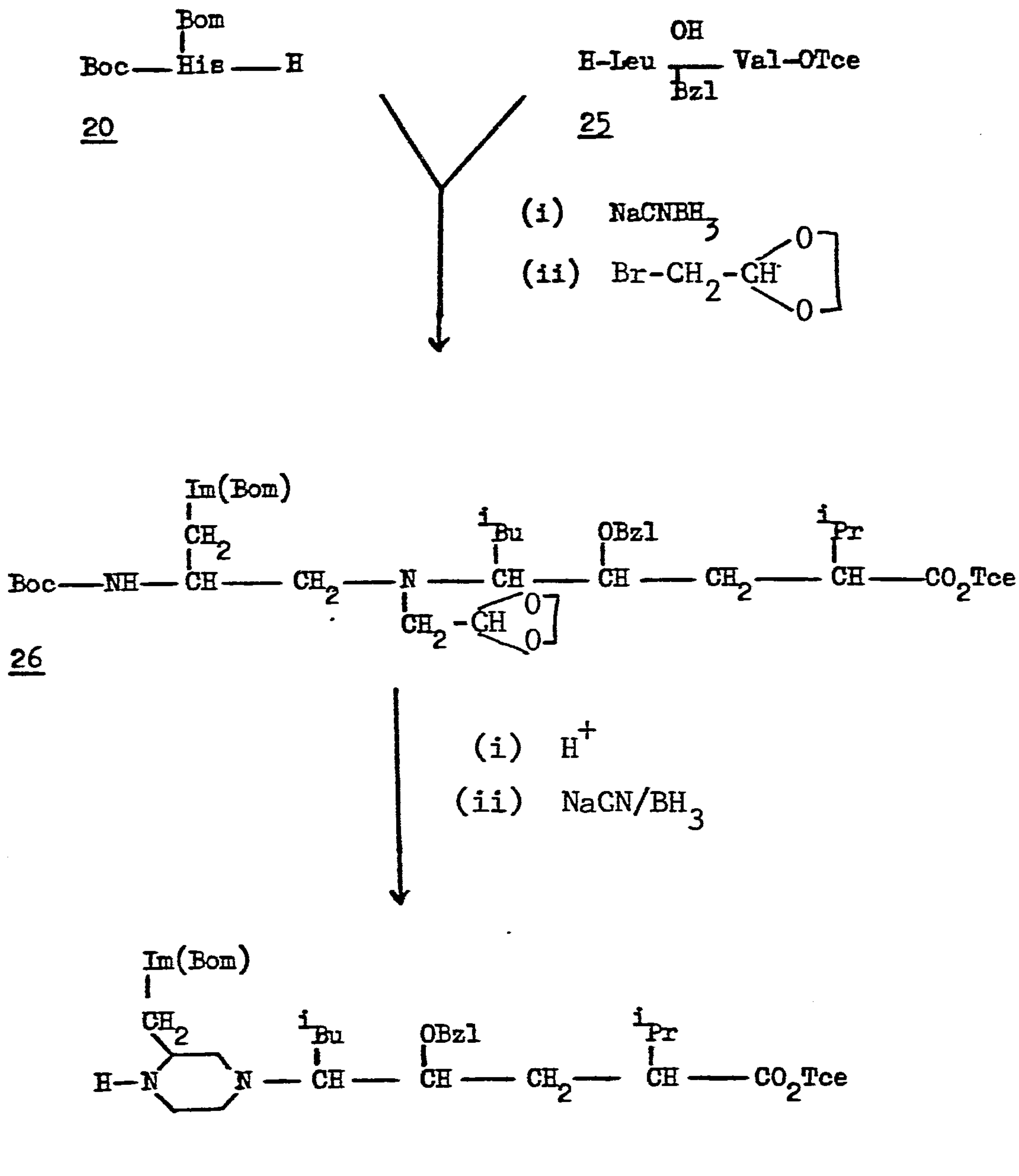


20

25

26

27

Compound 27 is synthesised as above then N-terminal Phe and C-terminal Ile$\overset{R}{-}$Phe residues are attached by the methods described herein giving, after deprotection, the compound of Example 26.

Scheme 9 (Example 27)

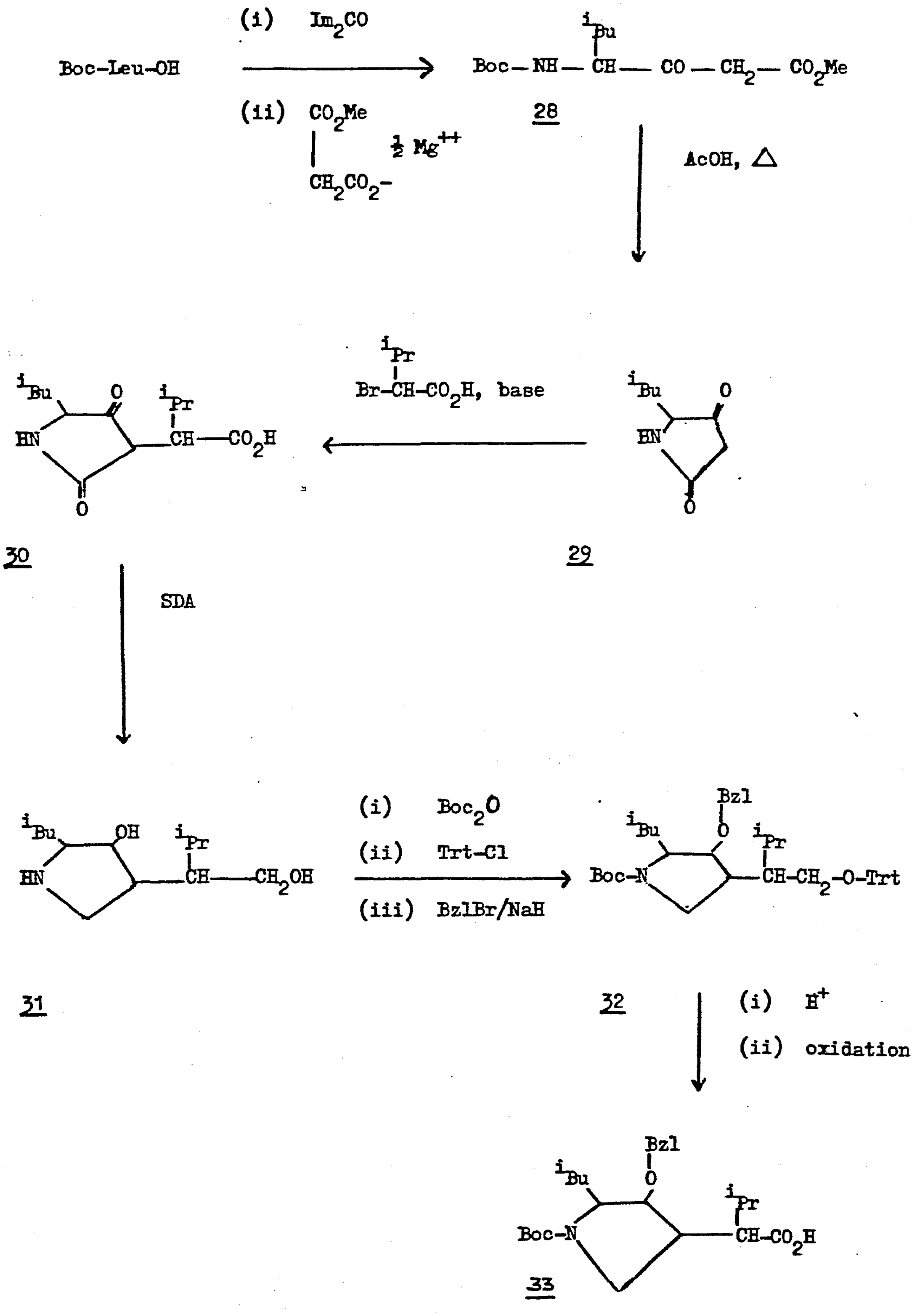


28

29

30

31

32

33

The compound 33 is synthesised as shown then N-terminal Phe-His and C-terminal Ile$\overset{Me}{-}$Phe residues attached by the methods described herein giving after deprotection the analogue of Example 27.

Scheme 10 (Example 28)

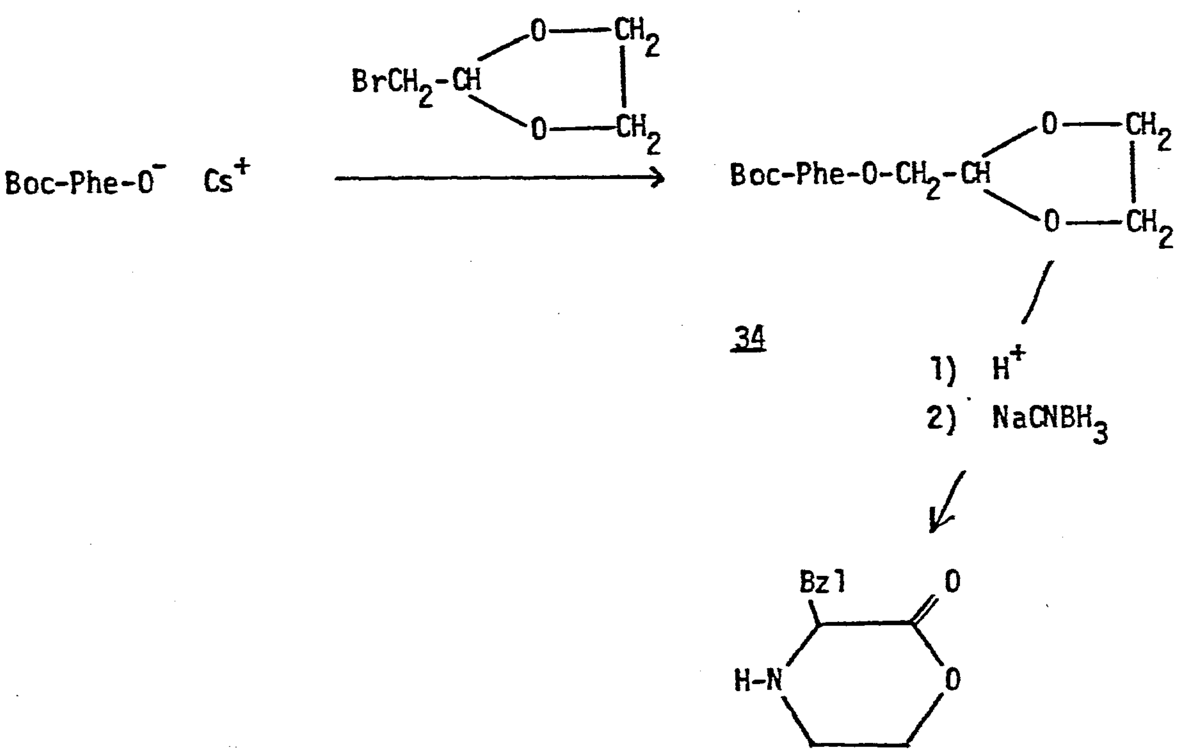


34

35

The Leu-OH-Val dipeptide analogue is made as given in detail earlier herein and the analogue of Example 28 made by the methods given, compound 35 giving the C-terminal and Phe-Abu the N-terminal residues.

ACTIVITY

The test results given herein are on the human renin/renin substrate reaction in vitro. The term is based on the methods described by J.A. Miller et al in Clinica Chimica Acta (1980) 101 5-15 and K. Poulsen and J. Jorgensen in J. Clin. Endocrinol. Metab. (1974) 39 816, and is based on the measurement, by radioimmunoassay, of angiotensin-I released from human renin substrate by human renin in human plasma. The inhibitor is dissolved in 0.01 N HCl (10 µl) and added to human plasma (75 µl) containing EDTA, and angiotensin-I antibody (15 µl) in 3M-Tris/HCl buffer (pH 6.9).

After incubation at 37°C for 0-120 minutes, the enzymic reaction is quenched by the addition of ice-cold 0.25M Tris/HCl buffer (pH 7.4) containing 0.01% of bovine serum albumin. 125I-labelled angiotensin-I is added, followed by equilibration at 4°C for 48 hours. Free and bound ligand are separated by the addition of dextran-coated charcoal, and the amount of bound radio-ligand determined in a gamma counter.

The results for the renin inhibitory activities of the present compounds thus tested are expressed as the $IC_{50}$, i.e. the molar concentration required to cause 50% inhibition. In many of the compounds of the invention very great potency, in the reduction of renin activity

remaining in the plasma in the presence of the analogue, is shown.

Detailed activity tests have been conducted only in animals but indicate corresponding activity in man confirmed by preliminary studies in volunteers.

In the in vivo studies compounds are infused into normal, conscious sodium-depleted dogs at rates of 0.01, 0.1, 1 and 10 mg/kg/hr. A maximum fall in boood pressure plasma renin (PR) angiotensin-I (AI) and angiotensin-II (AII) levels is obtained within 10 minutes at doses of 1 and 10 mg/kg/hr. When the infusion is stopped, blood pressure returns to baseline levels 30 minutes after the 1 mg/kg/hr. doses, but more slowly after the 10 mg/kg/hr. doses. Results of the same kind have been obtained in the baboon, in six animals, as a close model of man.

USES IN MAN

In use of the compounds, a person suffering from hypertension is for example given a nasal instillation preparation of 0.01 to 1 mg/kg body weight of the compound per dose, for example three times a day. Clinically significant maintained reduction of the hypertension is a positive indication of hypertension amenable to treatment by the method of the invention. The method may be similarly applied to patients in heart failure. In both the hypertension and the heart failure instances the plasma-renin may or may not be above normal.

Longer term, persons diagnosed as suffering from amenable hypertension as above are treated by means of a continuing course of one or more of the compounds.

Table 4

ABBREVIATIONS

| | |
|---|---|
| Abu | 2-Aminobutyryl |
| Boc | t-Butyloxycarbonyl |
| Bom | π -Benzyloxymethyl |
| $^{i}Bu$ | i-Butyl |
| $^{s}Bu$ | s-Butyl |
| $^{t}Bu$ | t-Butyl |
| Bzl | Benzyl |
| Im | Imidazol-4-yl |
| Leu $\overset{OH}{—}$ Val | Hydroxy isostere, $-CH(OH)CH_2-$ in place of -CONH- |
| Leu $\overset{K}{—}$ Val | Keto isostere, $-COCH_2-$ in place of -CONH- |
| Leu $\overset{Me}{—}$ Val | N-Methyl peptide, -CONMe- in place of -CONH- |
| Leu $\overset{NH_2}{—}$ Val | Amino isostere, $-CH(NH_2)CH_2-$ in place of -CONH- |
| Me | Methyl |
| Ms | Methylsulphonyl |
| NBS | N-Bromosuccinimide |
| Ph | Phenyl |
| Pht | Phthaloyl |
| $^{i}Pr$ | i-Propyl |
| SDA | Sodium dihydro-bis (2-methoxyethoxy)-aluminate |
| Tce | 2,2,2-Trichloroethyl |
| Thp | Tetrahydropyranyl |
| Troc | 2,2,2-Trichloro-ethoxycarbonyl |
| Trt | Triphenylmethyl |
| Ts | p-Toluenesulphonyl |
| Z | Benzyloxycarbonyl |

Table 4 (cont'd)

ABBREVIATIONS (cont'd)

| | |
|---|---|
| Ast | 3-Amino-3-deoxy-statine<br>$H_2N-CH(^iBu)-CH^*(NH_2)-CH_2-COOH$ * (R) or (s) |
| DMECI | N-dimethylaminopropyl-N'-diethyl carbodiimide |
| Pfp | Pentafluorophenyl |
| β Nal | 3-(2-naphthyl)-alanine<br>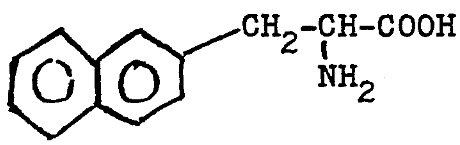 |
| α Nal | 3-(1-naphthyl)-alanine<br>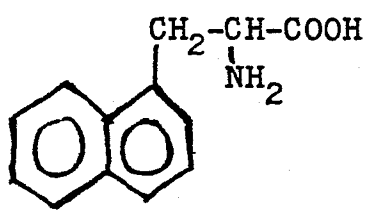 |

Further 'Pro' or 'proline', outside the formula of specific individual compounds, includes substituted (particularly - OH substituted) proline and its ring homologues azetidine carboxylic acid (one-$CH_2$-less) and piperidine carboxylic acid (one-$CH_2$-more).

CLAIMS (for states other than AUSTRIA)

1. Renin-inhibiting tetra-, penta- or hexapeptide analogues of formula

X-D-E—A—B—Z—W (XII)
8 9 10,11 12 13

where X and W are terminal groups; D, E, B and Z (of which any one or except with 'reduced' analogues any two may be absent) are aromatic, lipophilic or in the case of E aromatic lipophilic or basic amino acid or amino acid analogue residues; and A is an analogue of a lipophilic or aromatic dipeptide residue wherein the peptide link is replaced by a one- to four-atom carbon or carbon-nitrogen link which as such or in hydrated form is an unhydrolysable tetrahedral analogue of the transition state 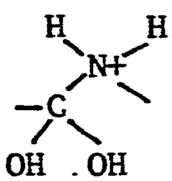 of the peptide bond.

2. Compounds of the general formula

X—D—E—A—B—Z—W (XIV)
8 9 10,11 12 13

where

X = H or an N-protecting group or groups, e.g. as follows:

(a) $R^3-(CH_2)_n-$, where n = 0-4, or

(b) $R^3$-CO- or

(c) $R^3$-O-CO- or

(d) $R^3-(CH_2)_n-CO-$ or )
(e) $R^3-(CH_2)_n-O-CO$ or ) where n = 0-5
(f) $R^3-O-(CH_2)_n-CO-$ or )

(g) $R^3SO_2-$ or

(h) $(R^3)_2-N-CO-$

In (a)-(h), $R^3$ = (i) H (except in (c) ) or
(ii) alkyl (e.g. t-butyl) or
(iii) cycloalkyl $C_{3-7}$ (e.g. cyclohexyl) or
(iv) bicycloalkyl or tricycloalkyl $C_{7-12}$ (e.g. isobornyl or adamantyl) or
(v) aryl (e.g. phenyl) or aryl alkyl

D = (a) absent
(b) aromatic amino acyl (e.g. Phe, $\alpha$Nal, $\beta$Nal, Tyr, His, Trp)
(c) lipophilic amino acyl (e.g. cyclohexyl-alanyl) (with (b) and (c) either as such or reduced at carbonyl)

E = (a) absent or

(b) aromatic amino acyl (e.g. His, Phe, Tyr, Trp) or

(c) lipophilic or basic amino acyl (e.g. 2-amino-butyryl, $\alpha$, $\delta$ -diaminovaleryl), (b) and (c) being as such or $N^{\alpha}$ -alkylated and/or reduced at carbonyl

A = (a) 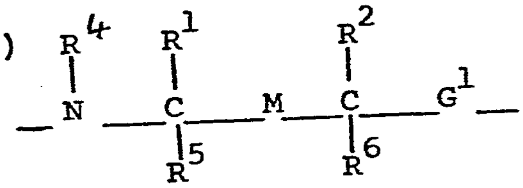 where

$R^4$, $R^5$ and $R^6$ the same or different =

(i) H or

(ii) alkyl (e.g. Me) or

(iii) $-(CH_2)_n-OH$ or $-(CH_2)_n-NH_2$

when n = 2, 3, 4

$G^1$ (and $G^2$ appearing below) =

(i) $-CH_2-(CH_2)_n-$ or

(ii) $-(CH_2)_n-CO-$ or

(iii) $-CO-(CH_2)_n-$

where n = 0-3

$R^1$ and $R^2$, the same or different =

(i) alkyl (e.g. $^iPr$, $^iBu$, $^sBu$) or

(ii) $ArCH_2$ or

(iii) other lipophilic group (e.g. cyclohexyl-methyl) or

(iv) H (especially for $R^2$)

M = (i) $-CH(OH)-(CH_2)_n-$ or

(ii) $-CH(NH_2)-(CH_2)_n-$ or

(iii) $-CH_2-(CH_2)_n-$ or

(iv) $-CO-(CH_2)_n-$ or

(v) $-(CH_2)_n-N(R^7)-$, where $R^7 = X$

(vi) $-CH(NH_2)-(CH_2)_n-CO-NH-$

where n = 0-2

with the provisos I) that when M = (i), (iii) or (iv) and n = 1 then two three or four, and when M = (v) and n = 1 then three or four, of D, E, B and Z are present and preferably that when M = (i), (iii) or (iv) then two, three or four and when M = (v) then three or four of said residues are present

II) when M = (i), (ii) or (iv) then $R^5$ and $R^6$ are H and when M = (iii)

or (v) then if one of $R^4$, $R^5$, $R^6$ and $R^7$ is said group $-(CH_2)_n-OH$ or $-(CH_2)_n-NH_2$ the others, the same or different, are H or alkyl

(b) 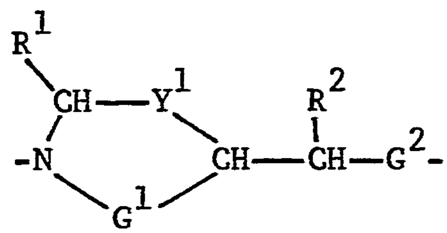 where

$R^1$, $R^2$ and $G^1$, $G^2$, the same or different, are as defined above and

$Y^1$ = (i) -CO- or

(ii) $-CH_2-$ or

(iii) -CH(OH)- or

(iv) $-CH(NH_2)-$ or

(v) $-CH_2-NR^3-$ ($R^3$ as above)

B = (a) absent or

(b) lipophilic or aromatic amino acyl (e.g. Val, Leu, Ile, Phe) either as such or $N^{\alpha}$-alkylated and/or reduced at carbonyl

Z = (a) absent or

(b) aromatic amino acyl (e.g. His, Phe, Tyr) or

(c) lipophilic amino acyl (e.g. cyclohexyl-alanyl), (b) and (c) being either as such or $N^{\alpha}$-alkylated and/or reduced at carbonyl ,

W = (a) -OH or other terminal group including those set out for W in general formula XV

(b) $-OR^3$ ) $R^3$ as above

(c) $-NH_2$, $-NHR^3$, $-NR_2^3$ )

(d) 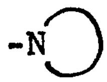 where N is part of a heterocyclic ring, preferably 5- or 6-membered, containing 1-3 heteroatoms (N, O or S) and of any degree of saturation and optionally substituted with $R^3$- or $R^3CH_2$-groups at one or more positions ($R^3$ as above)

<u>or</u>

Z-W = 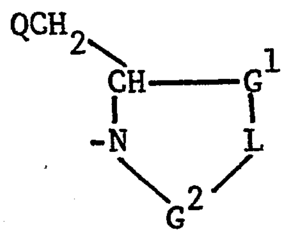 where L = (i) -NH- or

(ii) -O- or

(iii) $-NR^3$-

and $R^3$ and $G^1$ and $G^2$, the same or different, are as defined above and

Q = (i) H or

(ii) $C_{1-4}$ alkyl or

(iii) aryl or

(iv) imidazol-4-yl- or indol-3-yl

together with compounds in which, when one or more of the peptide bonds of the chain is represented by a 'reduced' isostere, the N atom of such isostere and the preceding or succeeding N atom in the chain are linked by a moiety as defined for $G^1$ and giving a five or six membered ring, and together further with compounds in which (except when M = (i), (iii), (iv) or (v), at least for n = 1) the above residues are present with further, N- or C- terminal,

aminoacyl or aminoacyl analogue residues(s) (particularly Pro or J-Pro interposed between X and D, 𝒥 being His or other basic or aromatic aminoacyl residue), and compounds (including such compounds with further residues) in which one or more remaining peptide links have been replaced by analogues M, all compounds being in free form or protected at one or more remaining peptide, carbonyl, amino or other reactive groups including peptide nitrogen.

3. The compounds of claim 2 wherein, in M, n = 1.

4. The compounds of claim 2 or 3, wherein M = $-CH(OH)-(CH_2)_n-$.

5. The compounds of claim 2 or 3, wherein M = $-CH(NH_2)-(CH_2)_n-$.

6. The compounds of claim 2 or 3, wherein M = $-CH(NH_2)-(CH_2)_n-CO-NH-$.

7. The compounds of claim 2 or 3, wherein A =

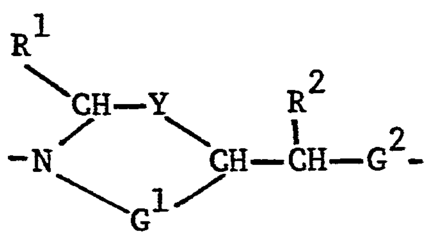

as defined herein.

8. The compounds of any of claims 2 to 7, wherein in A, $R^5$ and $R^6$ are hydrogen.

9. Compounds of the general formula:

X—Phe-His——A——B——Z—W (XV)

8 9 10,11 12 13

where Phe and His are optional (but are not both absent when A has the 'reduced' link below) and may further be in substituted form e.g. Phe by OH F Cl Br or Me, preferably at the 4-position, or His by Me; or Phe is replaced by Tyr, or His by spinacin

X = H; or an acyl or other N-protecting group, e.g. acetyl, pivaloyl, t-butyloxycarbonyl (Boc), benzoyl or lower alkyl (primarily $C_1$-$C_5$); or an L- or D- amino-acyl residue, which may itself be N-protected similarly and in particular may be Gly or D- or L- Pro, Val or Ile

A = 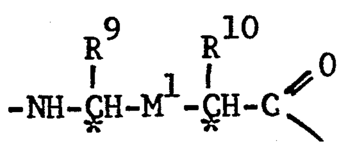 where $M^1$ is as M above or

particularly a 'reduced' 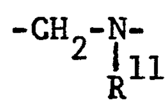 or

'keto' 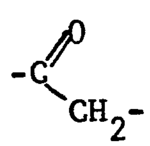 or 'hydroxy' $-CH(OH)-CH_2-$, or

'hydrocarbon' $-CH_2-CH_2-$ isostere bond

where the configuration at asymmetric centres is either R or S, where in the hydroxy isostere the hydroxy group may be present as such or protected

in ether $-OR^{12}$ or ester 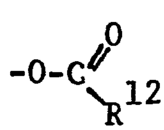 form where $R^{12}$

is as given under W below and where $R^9$ and $R^{10}$, the same or different = $^i$Pro (isopropyl), $^i$Bu (isobutyl), Bzl (benzyl) or other amino-acid side chain preferably lipophilic or aromatic;

$R^{11}$ = -H or an N-protecting group such as lower alkyl or lower acyl ($C_1$-$C_5$), or t-butyloxycarbonyl or benzyloxycarbonyl as such or ring substituted, or aryl sulphonyl e.g. $-SO_2Ph$ or $-SO_2-C_6H_4CH_3(p)$, or formyl;

B = D- or L- Val Leu or Ile or other D- or L- lipophilic amino-acyl residue;

Z = D- or L- Tyr, Phe, His or other L- or D- aromatic amino-acyl residue; and

W = (i) -OH as such or in protected ester form as $-OR^{12}$ where $R^{12}$ = lower alkyl primarily $C_1$-$C_5$ and particularly $^t$Bu, or cycloalkyl primarily $C_3$-$C_7$, or other ester forming group; or

(ii) $-NH_2$ as such or in protected amide form as $-NHR^{13}$ or $-N(R^{13})_2$ (where $R^{13}$ = an N-protecting or other substituent group e.g. lower alkyl as for $R^{12}$, and $(R^{13})_2$ = two such or e.g. cycloalkyl, primarily $C_3$-$C_7$) or as $-NH-(CH_2)_n-Q^1$ or $-NR^{13}-(CH_2)_n-Q^1$ (where n = 2 to 6 and $Q^1$ = $NH_2$ or

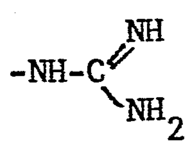 and wherein any of the hydrogens

attached to nitrogen may be substituted by $R^{13}$ or $(R^{13})_2$); or

(iii) an L- or D- serine or L- or D-lysine, arginine or other basic amino-acyl residue as such or in amide form, substituted amide form or ester form e.g. containing a group or groups as given for $R^{12}$ and $R^{13}$ above as the case may be; or

(iv) an amino alcohol residue derived therefrom as such or protected in ester or ether form e.g. containing a group as given for $R^{12}$ above;

or

Z + W = an alcohol derived from L- or D- Tyr, Phe, His or other L- or D- aromatic amino-acyl residue as such or protected in ester or ether form as above;

such compound being in the above form or modified by isosteric replacement of one or more remaining peptide bonds e.g. by reduced, $-CH_2-NH-$, keto, 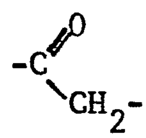, hydroxy, $-CH(OH)-CH_2-$, or hydrocarbon, $-CH_2-CH_2-$ or other analogue links M and further being in free form or in protected form at one or more remaining amino or amide (including peptide) nitrogen, carboxyl, hydroxy or other reactive groups, or in salt form at amino imidazole or carboxyl groups in particular as physiologically acceptable acid addition salts at basic centres.

10. The compounds of claim 9, wherein A contains the 'hydroxy' isostere bond.

11. Compounds as in claim 9 but having in place of A the group

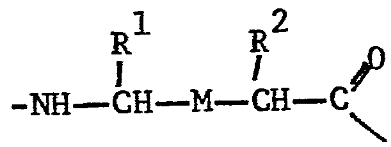

where M is as in claim 2 and $R^1$ and $R^2$ are as in claim 9.

12. The compounds of claim 11 wherein, in M, n = 1.

13. The compounds of claim 11 or 12, wherein M = $-CH(OH)-(CH_2)_n-$.

14. The compounds of claim 11 or 12 wherein M = $-CH(NH_2)-(CH_2)_n-CO-NH-$.

15. Compounds as in claim 9 but having in place of A the group

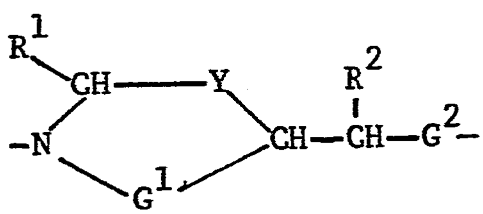

as defined in claim 2.

16. The compound

H-Leu$\overset{R}{\text{——}}$Val Ile His D-Lys-OH or

H-His Leu$\overset{R}{\text{——}}$Val Ile His D-Lys-OH

17. The compound

H-Leu$\overset{OH}{\text{——}}$Val Ile His Lys-OH or

Boc-Leu$\overset{OH}{\text{——}}$Val Ile His-OH or

H-Leu$\overset{OH}{\text{——}}$Val Ile His-OH or

Boc-His Leu$\overset{OH}{\text{——}}$Val Ile His-OH or

H-His Leu$\overset{OH}{\text{——}}$Val Ile His-OH or

Boc-Phe His Leu$\overset{OH}{\text{——}}$Val Ile His-OH or

H-Phe His Leu$\overset{OH}{\text{——}}$Val Ile His-OH or

Boc-Phe His Leu$\overset{OH}{\text{——}}$Gly Ile His-OH or

Boc-Phe His Leu$\overset{OH}{\text{——}}$Val Ile Phe-OMe or

Boc-Phe His Leu$\overset{OH}{\text{——}}$Val Ile-OMe or

Boc-Phe His Leu$\overset{OH}{\text{——}}$Val Ile His-OMe or

Boc-Phe His Leu$\overset{K}{\text{——}}$Val Ile His-OH or

Boc-Phe Ala Leu$\overset{OH}{\text{——}}$Val Ile His-OH or

Boc-Phe Phe Leu$\overset{OH}{\text{——}}$Val Ile His-OH

18. The compound

Boc-Phe His Leu$\overset{R}{\text{——}}$Val Ile His-OH

19. The compound

Boc-Phe His Ast$^{(R)}$ Ile His-OH or

Boc-Phe His Ast$^{(S)}$ Ile His-OH

20. The compound

Boc-Phe His Leu$\overset{NH_2}{\text{——}}$Val Ile His-$NH_2$ or

Boc-Phe His Leu$\overset{NH_2}{\text{——}}$Val Ile-NH-$CH_2CH_2$Ph or

Boc-Phe$\overset{Me}{\text{——}}$Phe Leu$\overset{NH_2}{\text{——}}$Val Ile-NH-$CH_2CH_2$-(2-pyridyl)

21. The compound

Boc-Phe His-N-CH($^i$Bu)-$CH_2$-NH-CH($^i$Pr)-CO-Ile His-OH

with $CH_2CH_2NH_2$ on N:

$$\text{His-}\underset{\displaystyle |\atop CH_2CH_2NH_2}{N}\text{-CH}$$

22. The compound

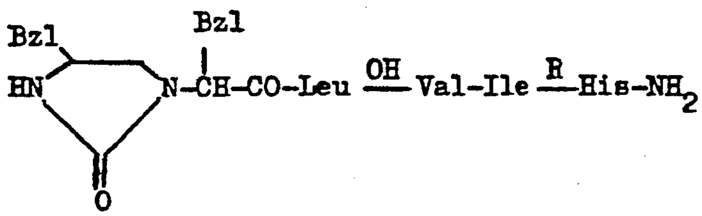

or

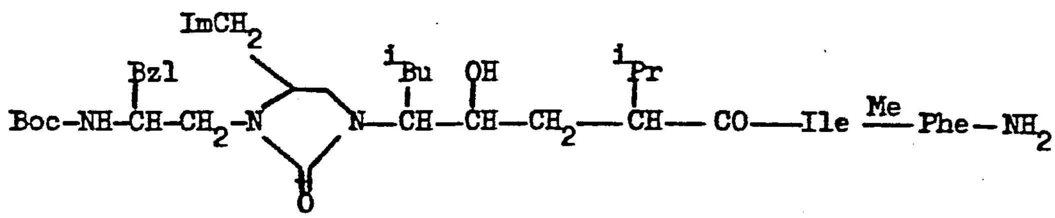

or

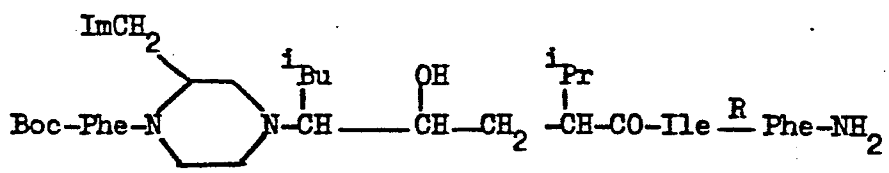

or

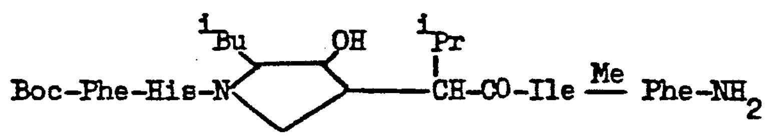

or

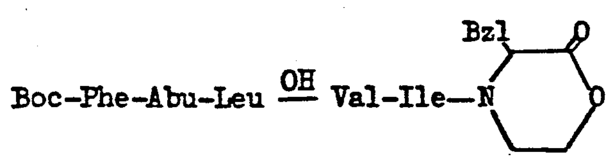

23. As such or in protected form the compound 3-amino-3-deoxy-statine:

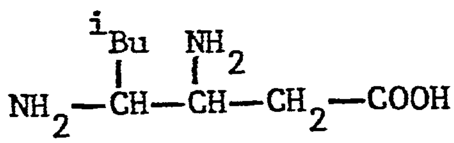

CLAIMS for AUSTRIA

24. A process for the preparation of compounds of formula set out in claim 1, or claim 2 and its appendant claims, or claim 9 and its appendant claims, or claims 16 to 22, wherein amino acyl or amino acyl analogue residues are successively reacted with each other, singly or as pre-synthesised units of two or more residues, until said compound has been built up.

25. A process for the preparation of the compound of formula set out in claim 23 wherein N-protected leucine is chain lengthened and the $NH_2$ substituent introduced to give said compound.

European Patent Office

# EUROPEAN SEARCH REPORT

0118223
Application number

EP 84 30 0733

## DOCUMENTS CONSIDERED TO BE RELEVANT

| Category | Citation of document with indication, where appropriate, of relevant passages | Relevant to claim | CLASSIFICATION OF THE APPLICATION (Int Cl. 3) |
|---|---|---|---|
| D,Y | EP-A-0 045 665 (PHILLIPS & LEIGH) * Whole document * | 1,2,4-6 | A 61 K 37/64<br>C 07 C 103/52 |
| P,Y | EP-A-0 081 783 (MERCK) * Whole document * | 1,2,4-6 | |
| Y | GB-A-2 058 077 (PHILLIPS & LEIGH) * Frontpage; page 9 * | 1,4-6 | |
| P,A | EP-A-0 082 568 (ANIC) * Frontpage; page 11; pages 1-3, claims * | 1 | |
| P,A | EP-A-0 097 994 (ANIC) * Whole document * | 1 | TECHNICAL FIELDS SEARCHED (Int. Cl. 3) |
| A | GB-A-2 091 270 (RICHTER GED.) * Whole document * | 1 | A 61 K 37/00<br>C 07 C 103/00 |

The present search report has been drawn up for all claims

| Place of search | Date of completion of the search | Examiner |
|---|---|---|
| THE HAGUE | 11-05-1984 | RAJIC M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

EPO Form 1503. 03.82